(19) Europäisches Patentamt
European Patent Office
Office européen des brevets

(11) EP 4 011 902 A1

(12) **EUROPEAN PATENT APPLICATION**
published in accordance with Art. 153(4) EPC

(43) Date of publication:
**15.06.2022 Bulletin 2022/24**

(21) Application number: **20863826.2**

(22) Date of filing: **09.09.2020**

(51) International Patent Classification (IPC):
***C07K 1/06*** *(2006.01)*

(52) Cooperative Patent Classification (CPC):
**C07K 1/06;** Y02P 20/55

(86) International application number:
**PCT/JP2020/034194**

(87) International publication number:
**WO 2021/049552 (18.03.2021 Gazette 2021/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.09.2019 JP 2019165967**

(71) Applicant: **FUJIFILM Corporation**
**Tokyo 106-8620 (JP)**

(72) Inventor: **YOSHIMITSU, Yuji**
**Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

# (54) METHOD FOR PRODUCING PEPTIDE COMPOUND, REAGENT FOR FORMING PROTECTING GROUP, AND HYDRAZINE DERIVATIVE

(57) Provided are a method for producing a peptide compound including a step of using a hydrazine derivative represented by Formula (1); a reagent for forming a protective group including the compound; and a hydrazine derivative. In Formula (1), a ring A represents an aromatic hydrocarbon ring or an aromatic heterocyclic ring, R represents a hydrogen atom, an amino protective group, an amino acid residue, or a peptide residue, k represents an integer of 1 to 5, n represents 1 or 2, and $R^A$'s each independently represent an aliphatic hydrocarbon group or an organic group having an aliphatic hydrocarbon group.

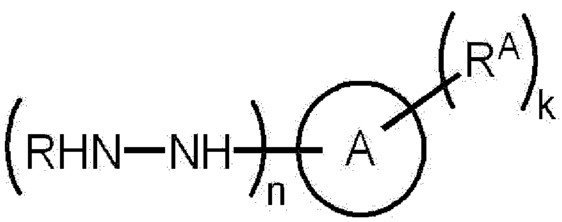

(1)

EP 4 011 902 A1

## Description

BACKGROUND OF THE INVENTION

1. Field of the Invention

The present disclosure relates to a method for producing a peptide compound, a reagent for forming a protective group, and a hydrazine derivative.

2. Description of the Related Art

Examples of a method for producing peptide include a solid phase method and a liquid phase method.

The solid phase method is advantageous in that isolation and purification after reaction can be performed by only washing resin. However, the solid phase method is associated with problems in that the reaction is essentially a heterogeneous phase reaction, a reaction agent or a reagent need to be used in excess to compensate for the low reactivity. In addition, tracing of the reaction and analysis of a reaction product supported by a carrier are difficult.

On the other hand, the liquid phase method is advantageous in that good reactivity is exhibited, and intermediate peptide can be purified by extraction and washing, isolation, and the like after a condensation reaction. However, the liquid phase method still has problems in each step of coupling reaction and deprotection.

As a separation carrier used for peptide synthesis in the related art, a separation carrier disclosed in WO2007/034812A has been known.

In addition, as a reagent for forming a protective group in the related art, a compound having a diphenylmethane structure, which is disclosed in WO2010/113939A, and a compound having a fluorene structure, which is disclosed in WO2010/104169A, have been known.

SUMMARY OF THE INVENTION

An object to be achieved by an embodiment of the present invention is to provide a method for producing a peptide compound having an excellent yield in a case of deprotection.

An object to be achieved by another embodiment of the present invention is to provide a reagent for forming a protective group having an excellent yield in a case of deprotection.

An object to be achieved by still another embodiment of the present invention is to provide a novel hydrazine derivative.

The methods for achieving the above-described objects include the following aspects.

<1> A method for producing a peptide compound, comprising: a step of using a hydrazine derivative represented by Formula (1).

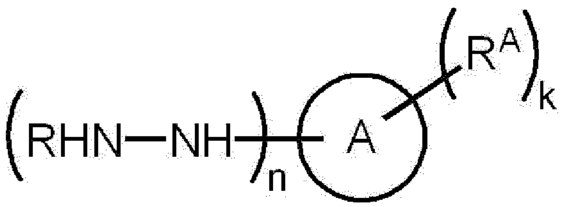

(1)

In Formula (1), a ring A represents an aromatic hydrocarbon ring or an aromatic heterocyclic ring, R represents a hydrogen atom, an amino protective group, an amino acid residue, or a peptide residue, k represents an integer of 1 to 5, n represents 1 or 2, $R^A$'s each independently represent an aliphatic hydrocarbon group or an organic group having an aliphatic hydrocarbon group, and at least one aliphatic hydrocarbon group in k pieces of $R^A$'s has 13 or more carbon atoms.

<2> The method for producing a peptide compound according to <1>,
in which the step of using the hydrazine derivative represented by Formula (1) is a C-terminal protecting step of protecting a carboxy group of an amino acid compound or a peptide compound with the hydrazine derivative represented by Formula (1).

<3> The method for producing a peptide compound according to <2>,
in which the amino acid compound or the peptide compound in the C-terminal protecting step is an N-terminal protected amino acid compound or an N-terminal protected peptide compound.

<4> The method for producing a peptide compound according to <3>, further comprising:

an N-terminal deprotecting step of deprotecting an N-terminal of an N-terminal and C-terminal protected amino acid compound or an N-terminal and C-terminal protected peptide compound, which is obtained in the C-terminal protecting step; and
a peptide chain extending step of condensing the N-terminal of a C-terminal protected amino acid compound or a C-terminal protected peptide compound, which is obtained in the N-terminal deprotecting step, with an N-terminal protected amino acid compound or an N-terminal protected peptide compound.

<5> The method for producing a peptide compound according to <4>, further comprising:
a precipitating step of precipitating the N-terminal and C-terminal protected peptide compound obtained in the peptide chain extending step.
<6> The method for producing a peptide compound according to <4>, further comprising:
an extracting step of adding water to the N-terminal and C-terminal protected peptide compound obtained in the peptide chain extending step to extract and separate impurities into a water phase.
<7> The method for producing a peptide compound according to <5> or <6>, further comprising: one or more times in the following order after the precipitating step or the extracting step,

a step of deprotecting the N-terminal of the obtained N-terminal and C-terminal protected peptide compound;
a step of condensing the N-terminal of the obtained C-terminal protected peptide compound with an N-terminal protected amino acid compound or an N-terminal protected peptide compound; and
a step of precipitating the obtained N-terminal and C-terminal protected peptide compound.

<8> The method for producing a peptide compound according to any one of <1> to <7>, further comprising:
a C-terminal deprotecting step of deprotecting a C-terminal protective group.
<9> The method for producing a peptide compound according to any one of <1> to <8>,
in which the ring A is a benzene ring, a naphthalene ring, or a pyridine ring.
<10> The method for producing a peptide compound according to any one of <1> to <9>,
in which, in Formula (1), at least two aliphatic hydrocarbon groups in k pieces of $R^A$'s have 13 or more carbon atoms.
<11> The method for producing a peptide compound according to any one of <1> to <10>,
in which n is 1.
<12> The method for producing a peptide compound according to any one of <1> to <11>,
in which k is 1.
<13> A reagent for forming a protective group comprising:
a hydrazine derivative represented by Formula (1a).

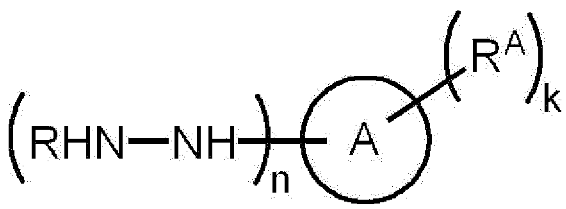
(1a)

In Formula (1a), a ring A represents an aromatic hydrocarbon ring or an aromatic heterocyclic ring, R represents a hydrogen atom or an amino protective group, k represents an integer of 1 to 5, n represents 1 or 2, $R^A$'s each independently represent an aliphatic hydrocarbon group or an organic group having an aliphatic hydrocarbon group, and at least one aliphatic hydrocarbon group in k pieces of $R^A$'s has 13 or more carbon atoms.
<14> The reagent for forming a protective group according to <13>,
in which the reagent for forming a protective group is a reagent for forming a protective group of a carboxy group.
<15> The reagent for forming a protective group according to <13> or <14>,
in which the reagent for forming a protective group is a C-terminal reagent for forming a protective group of a carboxy group of an amino acid compound, or of a peptide compound.
<16> The reagent for forming a protective group according to any one of <13> to <15>,
in which the ring A is a benzene ring, a naphthalene ring, or a pyridine ring.
<17> The reagent for forming a protective group according to any one of <13> to <16>,
in which, in Formula (1a), at least two aliphatic hydrocarbon groups in k pieces of $R^A$'s have 13 or more carbon atoms.
<18> The reagent for forming a protective group according to any one of <13> to <17>,

in which n is 1.
<19> The reagent for forming a protective group according to any one of <13> to <18>,
in which k is 1.
<20> A hydrazine derivative represented by Formula (1b).

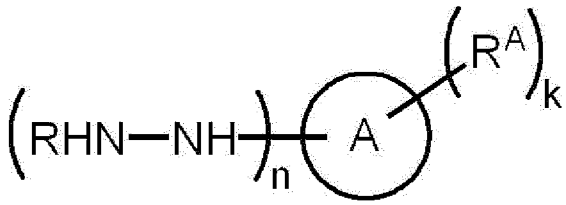

( 1b )

In Formula (1b), a ring A represents an aromatic hydrocarbon ring or an aromatic heterocyclic ring, R represents a hydrogen atom or an amino protective group, k represents an integer of 1 to 5, n represents 1 or 2, $R^A$'s each independently represent an aliphatic hydrocarbon group or an organic group having an aliphatic hydrocarbon group, and at least two aliphatic hydrocarbon groups in k pieces of $R^A$'s have 13 or more carbon atoms.
<21> The hydrazine derivative according to <20>,
in which the ring A is a benzene ring, a naphthalene ring, or a pyridine ring.
<22> The hydrazine derivative according to <20> or <21>,
in which n is 1.
<23> The hydrazine derivative according to any one of <20> to <22>,
in which k is 1.

According to an embodiment of the present invention, it is possible to provide a method for producing a peptide compound having an excellent yield in a case of deprotection.
In addition, according to another embodiment of the present invention, it is possible to provide a reagent for forming a protective group having an excellent yield in a case of deprotection.
In addition, according to still another embodiment of the present invention, it is possible to provide a novel hydrazine derivative.

DESCRIPTION OF THE PREFERRED EMBODIMENTS

Hereinafter, the contents of the present disclosure will be described in detail. The description of configuration requirements below is made based on representative embodiments of the present disclosure in some cases, but the present disclosure is not limited to such embodiments.
In addition, in the present specification, a numerical range represented using "to" means a range including numerical values described before and after the preposition "to" as a lower limit value and an upper limit value.
In numerical ranges described in stages in the present specification, an upper limit value or a lower limit value described in one numerical range may be replaced with an upper limit value or a lower limit value of a numerical range described in another stage. In addition, in the numerical ranges described in the present specification, the upper limit value or the lower limit value of the numerical ranges may be replaced with the values shown in examples.
In the present specification, the term "step" includes not only the independent step but also a step in which intended purposes are achieved even in a case where the step cannot be precisely distinguished from other steps.
In a case where substitution or unsubstitution is not noted in regard to the notation of a "group" (atomic group) in the present specification, the "group" includes not only a group not having a substituent but also a group having a substituent. For example, the concept of an "alkyl group" includes not only an alkyl group not having a substituent (unsubstituted alkyl group) but also an alkyl group having a substituent (substituted alkyl group).
In addition, a chemical structural formula in the present specification may be described by a simplified structural formula in which hydrogen atoms are omitted.
In the present disclosure, "% by mass" has the same definition as that for "% by weight", and "part by mass" has the same definition as that for "part by weight".
In addition, in the present disclosure, a combination of two or more preferred aspects is a more preferred aspect.
In the present specification, the "organic group having an aliphatic hydrocarbon group" is a monovalent (one bonding site) organic group having an aliphatic hydrocarbon group in its molecular structure.
The "aliphatic hydrocarbon group" is preferably a linear, branched, or cyclic saturated or unsaturated aliphatic hydrocarbon group having 5 or more carbon atoms, more preferably an aliphatic hydrocarbon group having 5 to 60 carbon atoms (also referred to as "the number of carbon atoms), still more preferably an aliphatic hydrocarbon group

having 5 to 40 carbon atoms, and particularly preferably an aliphatic hydrocarbon group having 10 to 30 carbon atoms.

The moiety of the aliphatic hydrocarbon group in the organic group having an aliphatic hydrocarbon group is not particularly limited, and may be present at the terminal (a monovalent group) or may be present at any other site (for example, a divalent group).

Examples of the "aliphatic hydrocarbon group" include a monovalent group such as an alkyl group, a cycloalkyl group, an alkenyl group, and an alkynyl group, a divalent group derived from these groups (divalent group obtained by removing one hydrogen atom from the monovalent group), and a group obtained by removing a hydroxy group from various steroid groups.

As the "alkyl group", for example, an alkyl group having 1 to 30 carbon atoms is preferable, and examples thereof include a methyl group, an ethyl group, a propyl group, an isopropyl group, a butyl group, a sec-butyl group, an isobutyl group, a tert-butyl group, a pentyl group, an isopentyl group, a 2-methylbutyl group, a 2-pentyl group, a 3-pentyl group, a hexyl Group, an octyl group, a 2-ethylhexyl group, a decyl group, a hexadecyl group, an octadecyl group, an icosyl group, a docosyl group, a tetracosyl group, a lauryl group, a tridecyl group, a myristyl group, and an isostearyl group.

As the alkyl group in the above-described aliphatic hydrocarbon group, an octadecyl group, an icosyl group, a docosyl group, or a tetracosyl group is preferable, and an icosyl group, a docosyl group, or a tetracosyl group is more preferable.

In addition, as the alkyl group in the above-described aliphatic hydrocarbon group, an alkyl group having 5 to 40 carbon atoms is preferable, and an alkyl group having 10 to 30 carbon atoms is more preferable.

In addition, the "alkyl group" in the present disclosure may be a linear alkyl group or a branched alkyl group, unless otherwise specified. Further, the alkenyl group and the alkynyl group are the same as the alkyl group.

As the "cycloalkyl group", for example, a cycloalkyl group having 3 to 30 carbon atoms, or the like is preferable, and examples thereof include a cyclopentyl group, a cyclohexyl group, an isobornyl group, and a tricyclodecanyl group. In addition, these may be linked repeatedly, or may be a fused-ring structure of two or more rings.

In addition, the cycloalkyl group in the above-described aliphatic hydrocarbon group, a cycloalkyl group having 5 to 30 carbon atoms is more preferable.

As the "alkenyl group", for example, an alkenyl group having 2 to 30 carbon atoms or the like is preferable, and examples thereof include a pentenyl group, a hexenyl group, and an oleyl group. In addition, as the "alkenyl group", an alkenyl group having 5 to 30 carbon atoms is more preferable.

As the "alkynyl group", for example, an alkynyl group having 2 to 30 carbon atoms or the like is preferable, and examples thereof include a 4-pentynyl group and a 5-hexenyl group. In addition, as the "alkynyl group", an alkynyl group having 5 to 30 carbon atoms is more preferable.

As the "steroid group", for example, a group having a cholesterol structure, a group having an estradiol structure, or the like is preferable.

As the "aryl group", for example, an aryl group having 6 to 14 carbon atoms or the like is preferable, and examples thereof include a phenyl group, a 1-naphthyl group, a 2-naphthyl group, a biphenylyl group, and a 2-anthryl group.

Examples of an "amino protective group (protective group of an amino group)" include a formyl group, an acyl group having 1 to 6 carbon atoms (for example, an acetyl group and a propionyl group), an alkoxycarbonyl group having 2 to 6 carbon atoms (for example, a methoxycarbonyl group, an ethoxycarbonyl group, and a tert-butoxycarbonyl group (Boc group)), a benzoyl group, an arylalkyloxycarbonyl group having 8 to 20 carbon atoms (for example, a benzyloxycarbonyl group and a 9-fluorenylmethoxycarbonyl group (Fmoc group)), a trityl group, a monomethoxytrityl group, a 1-(4,4-Dimethyl-2,6-dioxocyclohex-1-ylidene)-3-methylbutyl group, a phtaloyl group, an N,N-dimethylaminomethylene group, a silyl group (for example, a trimethylsilyl group, a triethylsilyl group, a dimethylphenylsilyl group, a tertbutyldimethylsilyl group, and a tert-butyldiethylsilyl group), and an alkenyl group having 2 to 6 carbon atoms. These groups may be substituted with one to three substituents selected from the group consisting of a halogen atom (for example, fluorine atom, chlorine atom, bromine atom, and iodine atom), an alkoxy group having 1 to 6 carbon atoms (for example, a methoxy group, an ethoxy group, and a propoxy group), and a nitro group.

As the amino protective group, an alkoxycarbonyl group having 1 to 6 carbon atoms or an arylalkyloxycarbonyl group having 7 to 20 carbon atoms is preferable.

Examples of a "protective group of a hydroxy group" include an alkyl group having 1 to 6 carbon atoms, a phenyl group, a trityl group, a formyl group, an acyl group having 1 to 6 carbon atoms, a benzoyl group, an arylalkylcarbonyl group having 7 to 10 carbon atoms, a 2-tetrahydropyranyl group, a 2-tetrahydrofuranyl group, a silyl group, and an alkenyl group having 2 to 6 carbon atoms. These groups may be substituted with one to three substituents selected from the group consisting of a halogen atom, an alkyl group having 1 to 6 carbon atoms, an alkoxy group having 1 to 6 carbon atoms, and a nitro group.

Examples of a "protective group of a carboxy group" include an alkyl group having 1 to 6 carbon atoms, an arylalkyl group having 7 to 10 carbon atoms, a phenyl group, a trityl group, a silyl group, and an alkenyl group having 2 to 6 carbon atoms. These groups may be substituted with one to three substituents selected from the group consisting

of a halogen atom, an alkoxy group having 1 to 6 carbon atoms, and a nitro group.

Examples of a "protective group of a carbonyl group" include cyclic acetals (for example, 1,3-dioxane) and acyclic acetals (for example, di(alkyl having 1 to 6 carbon atoms) acetal).

Examples of a "protective group of a guanidyl group" include a 2,2,4,6,7-pentamethyldihydrobenzofuran-5-sulfonyl group, a 2,3,4,5,6-pentamethylbenzenesulfonyl group, a tosyl group, and a nitro group.

Examples of a "protective group of a mercapto group (sulfhydryl group)" include a trityl group, a 4-methylbenzyl group, an acetylaminomethyl group, a t-butyl group, and a t-butylthio group.

(Method for producing peptide compound)

A method for producing a peptide compound according to an embodiment of the present disclosure includes a step of using a hydrazine derivative represented by Formula (1).

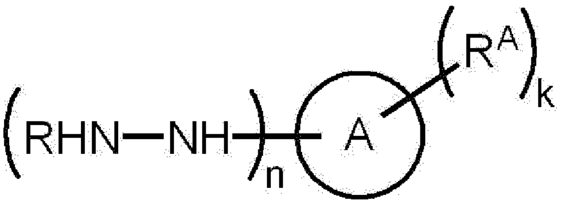

(1)

In Formula (1),

a ring A represents an aromatic hydrocarbon ring or an aromatic heterocyclic ring,
R represents a hydrogen atom, an amino protective group, an amino acid residue, or a peptide residue,
k represents an integer of 1 to 5,
n represents 1 or 2,
$R^A$'s each independently represent an aliphatic hydrocarbon group or an organic group having an aliphatic hydrocarbon group, and
at least one aliphatic hydrocarbon group in k pieces of $R^A$'s has 13 or more carbon atoms.

As a compound used as a protective group for amino acids or peptides in the related art, as described in WO2007/034812A, WO2010/113939A, and WO2010/104169A, the hydrazine derivative represented by Formula (1) has not been known at all.

Since the hydrazine derivative represented by Formula (1) has a hydrazino group bonded to an aromatic hydrocarbon ring or an aromatic heterocyclic ring, in a case of deprotection, it is possible to perform the deprotection by an oxidation reaction without setting the reaction system to be an acidic condition, it is possible to easily perform the deprotection in a homogeneous system, which is difficult in the related art, and yield of substitution reaction with a nucleophile is excellent. Therefore, it is presumed that the yield in a case of deprotection is excellent. In addition, because of high nucleophilicity of the hydrazine structure, loading yield (yield in a case of protection) is excellent, and since a bond with the C terminal of the peptide compound is a stable amide bond, it is presumed that by-production of a diketopiperazine compound described later can be suppressed.

In addition, in the hydrazine derivative represented by Formula (1), since at least one aliphatic hydrocarbon group in k pieces of $R^A$'s has 13 or more carbon atoms, after the condensation is completed, condensate can be precipitated and isolated by changing composition of the solvent, and yield of the obtained peptide compound is also excellent.

Furthermore, since the hydrazine derivative represented by Formula (1) according to the present disclosure has the above-described structure, it is possible to suppress side reactions and synthesize a peptide compound in a high yield.

Furthermore, by using the hydrazine derivative represented by Formula (1) according to the present disclosure, even poorly synthesized peptides such as unnatural peptide including unnatural amino acid, in which a side reaction is likely to occur, the peptide compound can be synthesized with high purity due to suppression of the side reaction. In particular, in a case where a diketopiperazine compound described later may be by-produced, the by-production of the diketopiperazine compound can be suppressed, and the peptide compound can be obtained in a high yield.

Hereinafter, the method for producing a peptide compound according to the embodiment of the present disclosure will be described in detail.

In the method for producing a peptide compound according to the embodiment of the present disclosure, the hydrazine derivative represented by Formula (1) can be used not only for formation of a protective group, but also for

denaturation of a peptide compound, adjustment of solubility in water or an organic solvent, improvement of crystallinity, multimerization, and the like.

Among these, the hydrazine derivative represented by Formula (1) is preferably used for formation of a protective group, and more preferably used for forming a C-terminal protective group in an amino acid compound or a peptide compound.

<Hydrazine derivative represented by Formula (1)>

The hydrazine derivative represented by Formula (1) in the present disclosure (hereinafter, also simply referred to as a "compound represented by Formula (1)") is shown below.

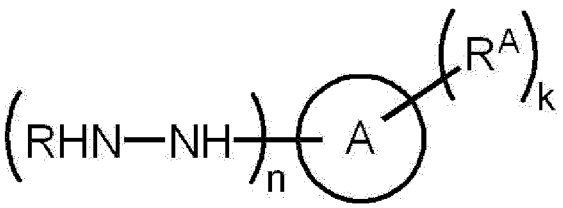

(1)

In Formula (1),

a ring A represents an aromatic hydrocarbon ring or an aromatic heterocyclic ring,
R represents a hydrogen atom, an amino protective group, an amino acid residue, or a peptide residue,
k represents an integer of 1 to 5,
n represents 1 or 2,
$R^A$'s each independently represent an aliphatic hydrocarbon group or an organic group having an aliphatic hydrocarbon group, and
at least one aliphatic hydrocarbon group in k pieces of $R^A$'s has 13 or more carbon atoms.

The ring A in Formula (1) represents an aromatic hydrocarbon ring or an aromatic heterocyclic ring, and the ring A may further have a substituent in addition to $R^A$ and -NH-NHR

The aromatic hydrocarbon ring and aromatic heterocyclic ring in the ring A may be a monocyclic ring or a fused ring having 2 or more rings. Examples of the above-described fused ring include a two or more fused polycyclic aromatic hydrocarbon ring, a two or more fused polycyclic aromatic heterocyclic ring, and a fused ring of one or more aromatic hydrocarbon rings and one or more aromatic heterocyclic rings.

From the viewpoint of deprotection rate, by-product inhibitory property of diketopiperazine compound, yield in a case of peptide compound synthesis, and yield in a case of deprotection, the ring A is more preferably a monocyclic ring or a bicyclic ring, and particularly preferably a monocyclic ring.

In addition, from the viewpoint of crystallization property, deprotection rate, and yield in a case of deprotection, the ring A is preferably an aromatic hydrocarbon ring.

As the ring A, from the viewpoint of deprotection rate, by-product inhibitory property of diketopiperazine compound, yield in a case of peptide compound synthesis, and yield in a case of deprotection, a benzene ring, a naphthalene ring, an anthracene ring, a phenanthrene ring, a tetracene ring, a triphenylene ring, a pyrene ring, a chrysene ring, a pyridine ring, a quinoline ring, an isoquinoline ring, a benzothiophene ring, a furan ring, a benzofuran ring, a pyrrole ring, an indole ring, a carbazole ring, a pyrazole ring, an indazole ring, or a thiophene ring is preferable, a benzene ring, a naphthalene ring, or a pyridine ring is more preferable, a benzene ring or a pyridine ring is still more preferable, and a benzene ring is particularly preferable.

Furthermore, as described later, the ring A may form a ring structure in which two or more substituents are bonded to each other, or the ring A may have a structure in which an aliphatic hydrocarbon ring, an aliphatic hetero ring, or the like is further fused.

The substituent which may be included in the ring A, in addition to $R^A$ and -NH-NHR, is not particularly limited, and examples thereof include an alkyl group having 1 to 10 carbon atoms (preferably having 1 to 4 carbon atoms), an alkoxy group having 1 to 10 carbon atoms, an aryloxy group having 6 to 20 carbon atoms, a halogen atom, a halogenated alkyl group having 1 to 10 carbon atoms, an aryl group having 6 to 20 carbon atoms, an acyl group having 1 to 20 carbon atoms, an acyloxy group having 6 to 20 carbon atoms, an alkoxycarbonyl group having 1 to 10 carbon atoms, an aryloxycarbonyl group having 6 to 20 carbon atoms, an alkylthio group having 6 to 20 carbon atoms, an arylthio group having 6 to 20 carbon atoms, $R^{st}$-CO-$NR^{st}$-, - CON$(R^{st})_2$, a dialkylamino group having 1 to 10 carbon atoms, an alkylarylamino group having 6 to 20 carbon atoms, a diarylamino group having 12 to 30 carbon atoms, and a group obtained

by combining two or more of these groups. $R^{st}$ represents a hydrogen atom, an alkyl group having 1 to 10 carbon atoms, or an aryl group having 6 to 20 carbon atoms.

As the amino protective group of R, the above-described amino protective group can be used, but an alkoxycarbonyl group having 2 to 6 carbon atoms or an arylalkyloxycarbonyl group having 8 to 20 carbon atoms is preferable.

The amino acid residue or peptide residue of R means a group in which a hydroxy group is removed from amino acid or peptide, which can be bonded to -NH-NH-. In addition, in a case where R is the amino acid residue or the peptide residue, the compound is also a protected amino acid compound or peptide compound. The types of the amino acid residue and the peptide residue are not particularly limited, but those described in Examples are preferable.

In addition, preferred examples of the amino acid residue include an amino acid residue described in a C-terminal protecting step described later, and preferred examples of the peptide residue include a residue obtained by removing a hydroxy group from a peptide compound described in the C-terminal protecting step described later.

Among these, as R, from the viewpoint of synthetic aptitude, a hydrogen atom, a Boc group, an Fmoc group, a benzyloxycarbonyl group (Cbz group), a 2,2,2-trichloroethoxycarbonyl group (Troc group), or an allyloxycarbonyl group (Alloc group) is preferable, and from the viewpoint of storage stability, a Boc group, an Fmoc group, a Cbz group, a Troc group, or an Alloc group is more preferable, a Boc group or an Fmoc group is still more preferable, and a Boc group is particularly preferable.

In addition, from the viewpoint of reactivity, a hydrogen atom is particularly preferable as R.

From the viewpoint of crystallization property and solubility in a solvent, k in Formula (1) is preferably an integer of 1 to 4, more preferably an integer of 1 to 3, and particularly preferably 1.

From the viewpoint of yield in a case of peptide compound synthesis and yield in a case of deprotection, n in Formula (1) is preferably 1.

$R^A$'s each independently represent an aliphatic hydrocarbon group or an organic group having an aliphatic hydrocarbon group, and at least one aliphatic hydrocarbon group in k pieces of $R^A$'s has 13 or more carbon atoms.

Among these, from the viewpoint of crystallization property and solubility, $R^A$'s are each independently preferably an organic group having an aliphatic hydrocarbon group, more preferably an organic group having two or more aliphatic hydrocarbon groups having 13 or more carbon atoms, still more preferably an organic group having two to six aliphatic hydrocarbon groups having 13 or more carbon atoms, and particularly preferably an organic group having two or three aliphatic hydrocarbon groups having 13 or more carbon atoms.

In addition, from the viewpoint of solubility in a solvent, crystallization property, and yield, the number of carbon atoms in the above-described aliphatic hydrocarbon group having 13 or more carbon atoms is preferably 13 or more, more preferably 14 or more, still more preferably 16 or more, and particularly preferably 18 or more.

The above-described organic group may be further substituted with a silyl group, a hydrocarbon group having a silyloxy structure, or an organic group having a perfluoroalkyl structure.

As the above-described silyl group, a trialkylsilyl group is preferable, and a silyl group having three alkyl groups having 1 to 6 carbon atoms is more preferable.

As the silyloxy structure in the above-described hydrocarbon group having a silyloxy structure, a trialkylsilyloxy structure is preferable, and a silyloxy structure having three alkyl groups having 1 to 6 carbon atoms is more preferable.

In addition, the above-described hydrocarbon group having a silyloxy structure preferably has 1 to 6 silyloxy structures.

Furthermore, the number of carbon atoms in the above-described hydrocarbon group having a silyloxy structure is preferably 10 or more, more preferably 10 to 100, and particularly preferably 16 to 50.

Preferred examples of the above-described hydrocarbon group having a silyloxy structure include a group represented by Formula (Si).

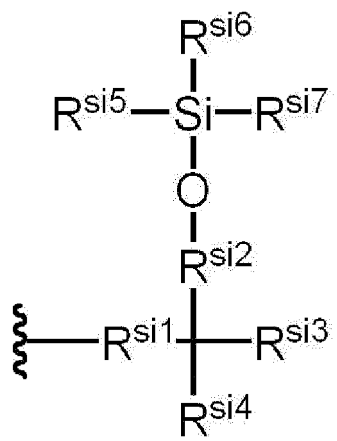

( Si )

In Formula (Si), $R^{si1}$ represents a single bond or an alkylene group having 1 to 3 carbon atoms, $R^{si2}$ represents an alkylene group having 1 to 3 carbon atoms, $R^{si3}$ and $R^{si4}$ each independently represent a hydrogen atom, an alkyl group having 1 to 6 carbon atoms, or - $OSiR^{si5}R^{si6}R^{si7}$, and $R^{si5}$ to $R^{si7}$ each independently represent an alkyl group having 1 to 6 carbon atoms or an aryl group.

$R^{si5}$ to $R^{si7}$ in Formula (Si) are each independently preferably an alkyl group having 1 to 6 carbon atoms or a phenyl group, and more preferably an alkyl group having 1 to 6 carbon atoms.

As the perfluoroalkyl structure in the above-described organic group having a perfluoroalkyl structure, a perfluoroalkyl structure having 1 to 20 carbon atoms is preferable, a perfluoroalkyl structure having 5 to 20 carbon atoms is more preferable, and a perfluoroalkyl structure having 7 to 16 carbon atoms is particularly preferable. In addition, the above-described perfluoroalkyl structure may be linear, may have a branch, or may have a ring structure.

The above-described organic group having a perfluoroalkyl structure is preferably a perfluoroalkyl group, an alkyl group having a perfluoroalkyl structure, or an alkyl group having a perfluoroalkyl structure and an amide bond in the alkyl chain.

The number of carbon atoms in the above-described organic group having a perfluoroalkyl structure is preferably 5 or more, more preferably 10 or more, still more preferably 10 to 100, and particularly preferably 16 to 50.

Preferred examples of the above-described organic group having a perfluoroalkyl structure include groups shown below.

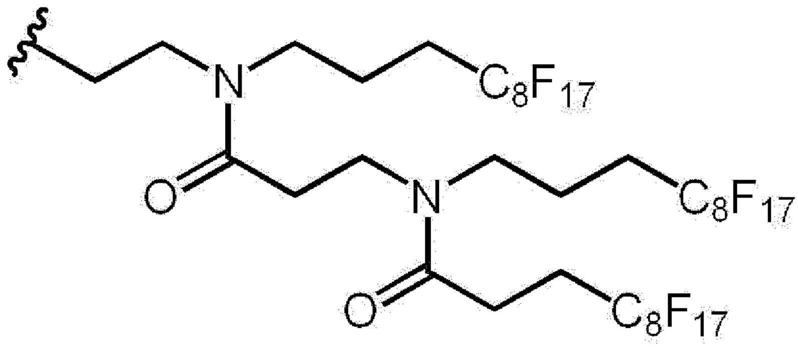

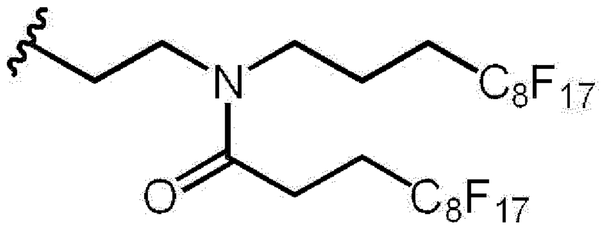

A moiety other than the aliphatic hydrocarbon group in the organic group having an aliphatic hydrocarbon group can be optionally set. For example, the above-described organic group may have a moiety such as an ether bond, a thioether bond, an ester bond, a sulfonamide bond, an amide bond, and a hydrocarbon group (monovalent group or divalent group) other than the "aliphatic hydrocarbon group".

Examples of the hydrocarbon group other than the aliphatic hydrocarbon group include an aliphatic hydrocarbon group having 1 to 4 carbon atoms and an aromatic hydrocarbon group, and specifically, for example, a monovalent group such as an alkyl group having 1 to 4 carbon atoms and an aryl group or a divalent group derived from the monovalent group is used. Among these, an aryl group having 6 to 10 carbon atoms is more preferable, and a phenyl group is particularly preferable.

In addition, the above-described aliphatic hydrocarbon group and the hydrocarbon group other than the above-described aliphatic hydrocarbon group may be substituted with a substituent selected from a halogen atom, an oxo group, and the like.

In $R^A$, the bond (substitution) of the organic group having an aliphatic hydrocarbon group to the ring A may be through the above-described aliphatic hydrocarbon group or the above-described hydrocarbon group existing in $R^A$, that is, may be directly bonded by a carbon-carbon bond, or may be through the moiety such as an ether bond, a thioether bond, an ester bond, an amide bond, and a sulfonamide bond, which exists in $R^A$. Among these, from the viewpoint of ease of synthesizing compound, the organic group having an aliphatic hydrocarbon group preferably has at least an ester bond, an amide bond, or a sulfonamide bond, and more preferably has at least an ester bond or an amide bond.

In addition, from the viewpoint of synthetic aptitude, crystallization property, and solubility, the above-described organic group having an aliphatic hydrocarbon group is preferably a group represented by Formulae (A1) to (A5), more preferably a group represented by Formulae (A1) to (A4), still more preferably a group represented by Formula (A1) or Formula (A2), and particularly preferably a group represented by Formula (A1).

In addition, from the viewpoint of crystallization property and solubility, the group represented by Formula (A5) is preferably a group represented by Formula (A6) or Formula (A7).

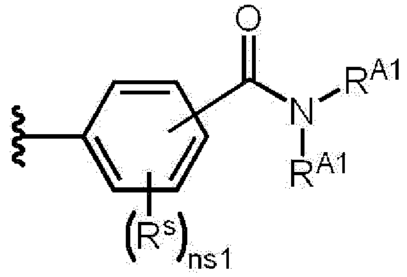

( A1 )

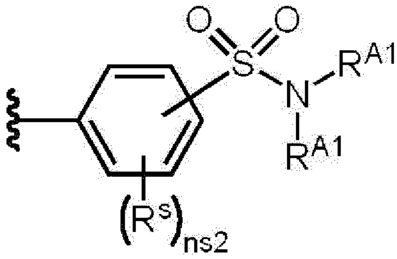

( A2 )

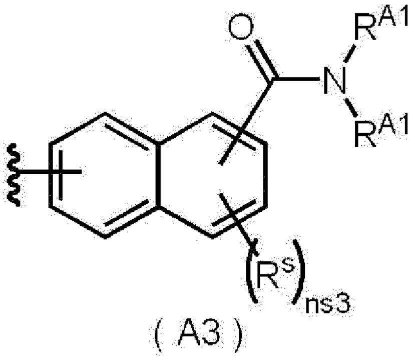

( A3 )

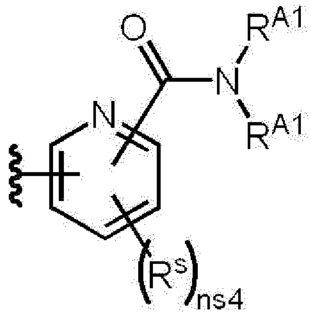

( A4 )

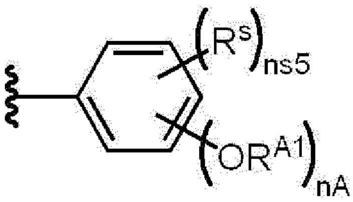

( A5 )

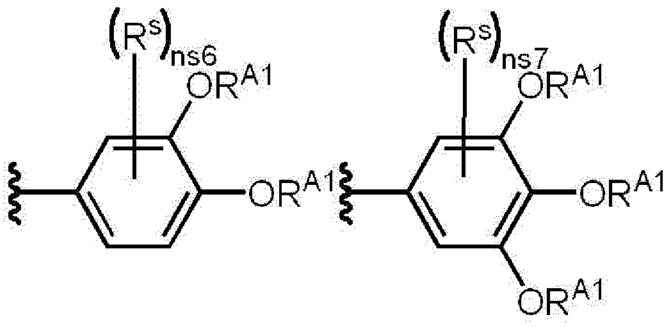

( A6 ) ( A7 )

In Formulae (A1) to (A7), $R^{A1}$'s each independently represent an aliphatic hydrocarbon group having 13 or more carbon atoms, nA represents an integer of 1 to 5, $R^s$'s each independently represent a substituent, ns1 and ns2 each independently represent an integer of 0 to 4, ns3 represents an integer of 0 to 6, ns4 represents an integer of 0 to 3, ns5 represents an integer of 0 to (5-nA), ns6 represents an integer of 0 to 3, ns7 represents an integer of 0 to 2, and a wavy line portion represents a bonding position with other structures.

From the viewpoint of yield in a case of peptide compound synthesis and availability of raw materials, $R^{A1}$'s in Formulae (A1) to (A7) are each independently preferably an aliphatic hydrocarbon group having 16 to 40 carbon atoms, more preferably an aliphatic hydrocarbon group having 18 to 36 carbon atoms, still more preferably an alkyl group having 18 to 32 carbon atoms, and particularly preferably a linear alkyl group having 18 to 22 carbon atoms.

In addition, from the viewpoint of synthetic aptitude, $R^{A1}$'s in Formulae (A1) to (A7) are preferably all the same group.

From the viewpoint of solubility and crystallization property, nA in Formula (A5) is preferably an integer of 1 to 4, more preferably an integer of 2 to 4, and particularly preferably 2 or 3.

Preferred examples of $R^s$ in Formulae (A1) to (A7) include the same substituents as the above-described "substituent which may be included in the ring A".

ns1 and ns2 in Formula (A1) or Formula (A2) are each independently preferably an integer of 0 to 2, more preferably 0 or 1, and particularly preferably 0.

ns3 in Formula (A3) is preferably an integer of 0 to 4, more preferably an integer of 0 to 2, and particularly preferably 0.

ns4 in Formula (A4) is preferably an integer of 0 to 2, more preferably 0 or 1, and particularly preferably 0.

ns5 to ns7 in Formulae (A5) to (A7) are each independently preferably 0 or 1, and more preferably 0.

In the hydrazine derivative represented by Formula (1), from the viewpoint of solubility in a solvent, crystallization property, deprotection rate, by-product inhibitory property of diketopiperazine compound, yield in a case of peptide compound synthesis, and yield in a case of deprotection, the total number of carbon atoms in all aliphatic hydrocarbon groups included in all $R^A$'s is preferably 26 or more, more preferably 26 to 200, still more preferably 32 to 100, particularly preferably 34 to 80, and most preferably 44 to 66.

In addition, the hydrazine derivative represented by Formula (1) is a compound which has at least one aliphatic hydrocarbon group having 13 or more carbon atoms in k pieces of $R^A$'s, and from the viewpoint of solubility in a solvent and crystallization property, it is preferable that at least two aliphatic hydrocarbon groups in k pieces of $R^A$'s have 13 or more carbon atoms, and it is more preferable to have one or more $R^A$'s having two or more aliphatic hydrocarbon groups having 13 or more carbon atoms.

For example, in a case where "at least two aliphatic hydrocarbon groups in k pieces of $R^A$'s have 13 or more carbon atoms", the hydrazine derivative represented by Formula (1) may have an aspect having one or more $R^A$'s having two or more aliphatic hydrocarbon groups having 13 or more carbon atoms, may have an aspect having two or more $R^A$'s having only one aliphatic hydrocarbon group having 13 or more carbon atoms, or may have both aspects.

Furthermore, from the viewpoint of solubility and crystallization property, the hydrazine derivative represented by Formula (1) is more preferably a compound having at least one aliphatic hydrocarbon group having 13 to 100 carbon atoms, still more preferably a compound having at least one aliphatic hydrocarbon group having 18 to 40 carbon atoms, and particularly preferably a compound having at least one aliphatic hydrocarbon group having 20 to 36 carbon atoms.

In the hydrazine derivative represented by Formula (1), the above-described effects are more excellent in a case where the number of carbon atoms in the at least one aliphatic hydrocarbon group in k pieces of $R^A$'s is preferably 14 or more, more preferably 16 or more, still more preferably 18 or more, and particularly preferably 20 or more. The reason is considered that, as the number of carbon atoms increases, the contribution ratio of hydrophobicity in the entire molecule increases, which makes it easier to dissolve in a hydrophobic solvent, and with regard to a hydrophilic solvent, presumed that, as the number of carbon atoms increases, the cohesive force increases, which makes it easier to be crystallized.

Furthermore, from the viewpoint of crystallization property, the above-described aliphatic hydrocarbon group is preferably an alkyl group and more preferably a linear alkyl group.

In addition, from the viewpoint of solubility in a solvent and crystallization property, the number of carbon atoms in one $R^A$ is preferably 13 to 200, more preferably 18 to 150, still more preferably 18 to 100, and particularly preferably 20 to 80, respectively.

In Formula (1), from the viewpoint of solubility in a solvent, crystallization property, and yield, it is preferable that at least one $R^A$ is a group represented by any of Formula (f1), Formula (a1), Formula (b1), or Formula (e1), it is more preferable to be a group represented by Formula (f1) or Formula (a1), and it is particularly preferable to be a group represented by Formula (f1).

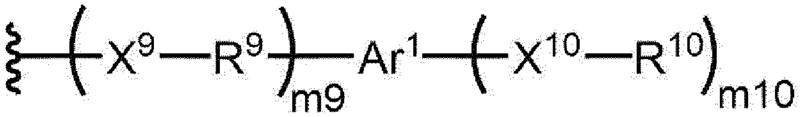

( f1 )

In Formula (f1), a wavy line portion represents a bonding position to other structures, m9 represents an integer of 1 to 3, $X^9$'s each independently represent a single bond, -O-, -S-, - COO-, -OCO-, -OCONH-, -NHCONH-, -NHCO-, or -CONH-, $R^9$'s each independently represent a divalent aliphatic hydrocarbon group, $Ar^1$ represents an (m10+1)-valent aromatic group or an (m10+1)-valent heteroaromatic group, m10 represents an integer of 1 to 3, $X^{10}$'s each independently represent a single bond, -O-, -S-, -COO-, -OCO-, -OCONH-, -NHCONH-, -NHCO-, or -CONH-, $R^{10}$'s each independently represent a monovalent aliphatic hydrocarbon group, and at least one of $R^{10}$'s is a monovalent aliphatic hydrocarbon group having 5 or more carbon atoms.

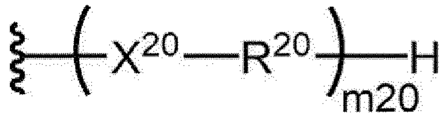

( a1 )

In Formula (a1), a wavy line portion represents a bonding position to other structures, m20 represents an integer of 1 to 10, $X^{20}$'s each independently represent a single bond, -O-, -S-, -COO-, -OCO-, -OCONH-, -NHCONH-, -NHCO-, or -CONH-, $R^{20}$'s each independently represent a divalent aliphatic hydrocarbon group, and at least one of $R^{20}$'s is a divalent aliphatic hydrocarbon group having 5 or more carbon atoms.

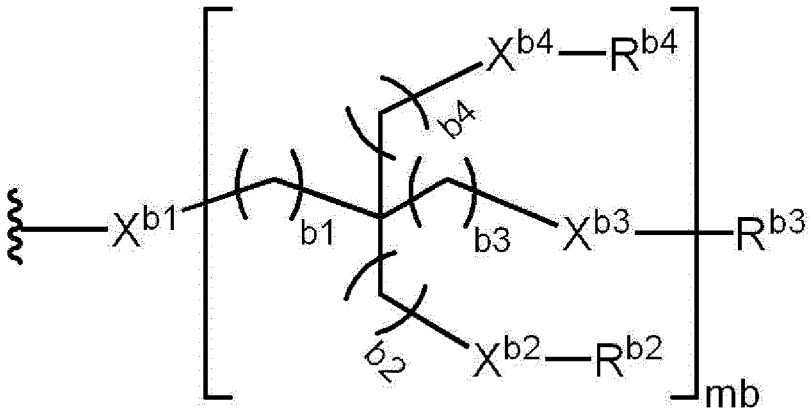

( b1 )

In Formula (b1), a wavy line portion represents a bonding position to other structures, mb represents 1 or 2, b1 to b4 each independently represent an integer of 0 to 2, $X^{b1}$ to $X^{b4}$ each independently represent a single bond, -O-, -S-, -COO-, -OCONH-, or -CONH-, $R^{b2}$ and $R^{b4}$ each independently represent a hydrogen atom, a methyl group, or an aliphatic hydrocarbon group having 5 or more carbon atoms, and $R^{b3}$ represents an aliphatic hydrocarbon group having 5 or more carbon atoms.

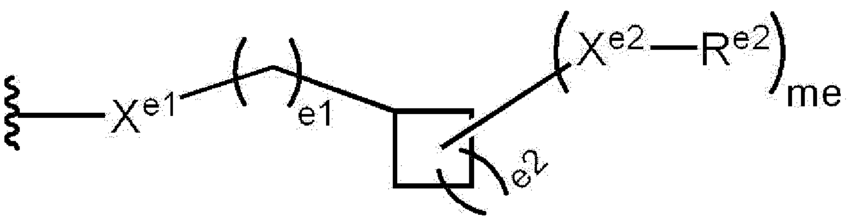

( e1 )

In Formula (e1), a wavy line portion represents a bonding position to other structures, $X^{e1}$ represents a single bond, -O-, -S-, -NHCO-, or -CONH-, me represents an integer of 0 to 15, e1 represents an integer of 0 to 11, e2 represents an integer of 0 to 5, $X^{e2}$'s each independently represent a single bond, -O-, -S-, -COO-, -OCONH-, -NHCO-, or -CONH-, and $R^{e2}$'s each independently represent a hydrogen atom, a methyl group, or an organic group having an aliphatic hydrocarbon group having 5 or more carbon atoms.

m9 in Formula (f1) is preferably 1 or 2 and more preferably 1.

$X^9$ and $X^{10}$ in Formula (f1) are each independently preferably -O-, -S-, -COO-, - OCONH-, or -CONH-, and more preferably -O-.

$R^9$'s in Formula (f1) are each independently preferably an alkylene group having 1 to 10 carbon atoms, more preferably an alkylene group having 1 to 4 carbon atoms, and particularly preferably a methylene group.

$R^{10}$'s in Formula (f1) are each independently preferably a monovalent aliphatic hydrocarbon group having 5 to 60 carbon atoms, more preferably a monovalent aliphatic hydrocarbon group having 12 to 50 carbon atoms, still more preferably a monovalent aliphatic hydrocarbon group having 18 to 40 carbon atoms, and particularly preferably a monovalent aliphatic hydrocarbon group having 20 to 32 carbon atoms. In addition, $R^{10}$'s are each independently preferably a linear alkyl group or a branched alkyl group and more preferably a linear alkyl group.

m10 in Formula (f1) is preferably 2 or 3 and more preferably 2.

$Ar^1$ in Formula (f1) is preferably an (m10+1)-valent aromatic group, more preferably a group obtained by removing (m10+1) pieces of hydrogen atoms from benzene or a group obtained by removing (m10+1) pieces of hydrogen atoms from naphthalene, and particularly preferably a group obtained by removing (m10+1) pieces of hydrogen atoms

from benzene.

In addition, from the viewpoint of solubility in a solvent, crystallization property, and yield, the group represented by Formula (f1) is preferably a group represented by Formula (f2).

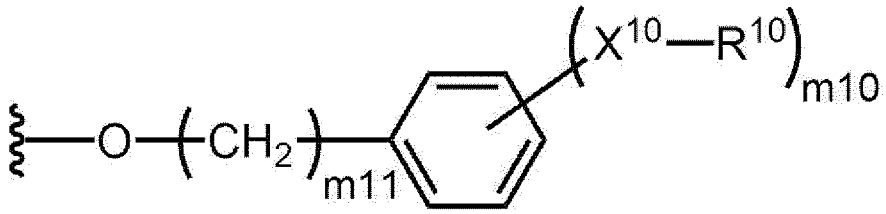

(f2)

in Formula (f2), a wavy line portion represents a bonding position to other structures, m10 represents an integer of 1 to 3, m11 represents an integer of 1 to 3, $X^{10}$'s each independently represent a single bond, -O-, -S-, -COO-, -OCO-, -OCONH-, -NHCONH-, -NHCO-, or -CONH-, and $R^{10}$'s each independently represent a monovalent aliphatic hydrocarbon group having 5 or more carbon atoms.

m10, $X^{10}$, and $R^{10}$ in Formula (f2) have the same meaning as m10, $X^{10}$, and $R^{10}$ in Formula (f1), respectively, and the preferred aspects thereof are also the same.

m11 in Formula (f2) is preferably 1 or 2 and more preferably 1.

m20 in Formula (a1) is preferably 1 or 2.

$X^{20}$'s in Formula (a1) are each independently preferably -O-, -S-, -COO-, -OCONH-, or -CONH-, and more preferably -O-.

$R^{20}$ in Formula (a1) is preferably a divalent aliphatic hydrocarbon group having 5 or more carbon atoms, more preferably a divalent aliphatic hydrocarbon group having 5 to 60 carbon atoms, still more preferably a divalent aliphatic hydrocarbon group having 8 to 40 carbon atoms, and particularly preferably a divalent aliphatic hydrocarbon group having 12 to 32 carbon atoms. In addition, $R^{20}$ is preferably a linear alkylene group.

mb in Formula (b1) is preferably 1.

b1 to b4 in Formula (b1) are each independently preferably 1 or 2 and more preferably 1.

$X^{b1}$ to $X^{b4}$ in Formula (b1) are each independently preferably -O-, -S-, -COO-, - OCONH-, or -CONH-, and more preferably -O-.

$R^{b2}$ and $R^{b4}$ in Formula (b1) are each independently preferably a hydrogen atom, a methyl group, or an aliphatic hydrocarbon group having 5 to 60 carbon atoms, more preferably a hydrogen atom, a methyl group, or an alkyl group having 8 to 40 carbon atoms, and particularly preferably a hydrogen atom, a methyl group, or an alkyl group having 12 to 32 carbon atoms.

$R^{b3}$ in Formula (b1) is preferably a monovalent aliphatic hydrocarbon group having 5 to 60 carbon atoms, more preferably a monovalent aliphatic hydrocarbon group having 5 to 60 carbon atoms, still more preferably a monovalent aliphatic hydrocarbon group having 8 to 40 carbon atoms, and particularly preferably a monovalent aliphatic hydrocarbon group having 12 to 32 carbon atoms. In addition, $R^{b3}$ is preferably a linear alkyl group.

In addition, in the compound represented by Formula (1) according to the present disclosure, from the viewpoint of solubility in a solvent, preferred examples of the above-described organic group having an aliphatic hydrocarbon group include an organic group having an aliphatic hydrocarbon group having a branch, and more preferred examples thereof include groups shown below. A wavy line portion represents a bonding position to another structure, nt2 represents an integer of 3 or more, and nt3 represents an integer set such that the total number of carbon atoms in the following group is 14 to 300.

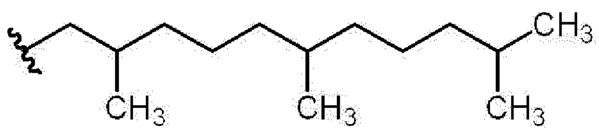

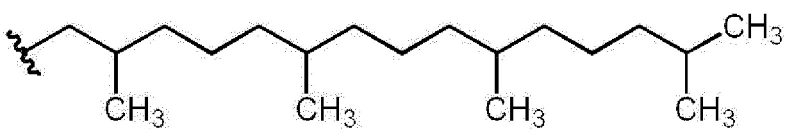

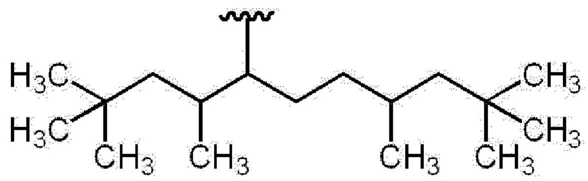

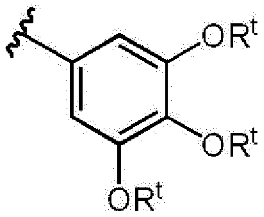

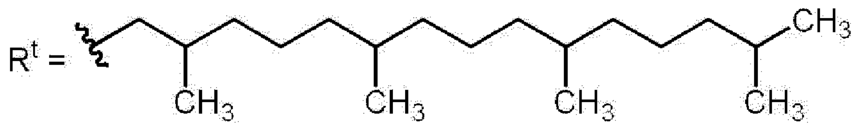

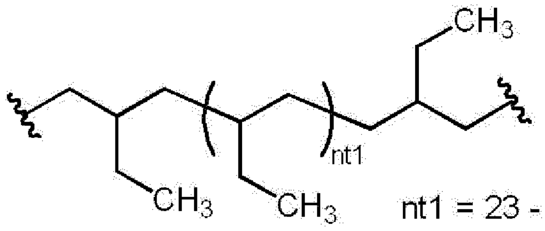

nt1 = 23 - 34

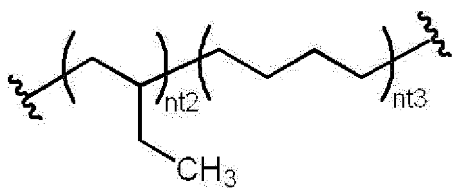

In addition, from the viewpoint of synthetic aptitude and yield in a case of deprotection, the hydrazine derivative represented by Formula (1) is preferably a compound represented by any of Formulae (B1) to (B5), more preferably a compound represented by any of Formulae (B1) to (B4), and particularly preferably a compound represented by Formula (B1).

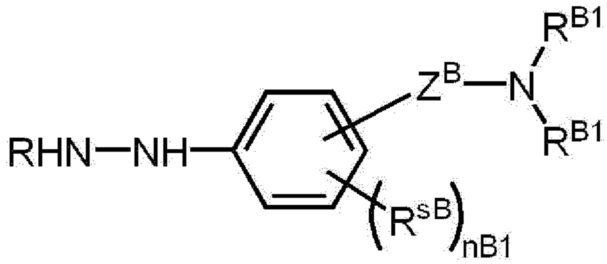

( B1 )

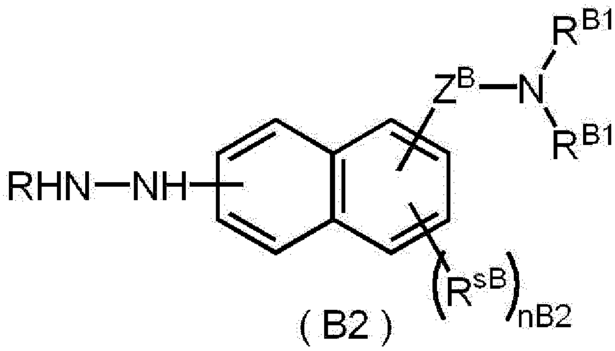

(B2)

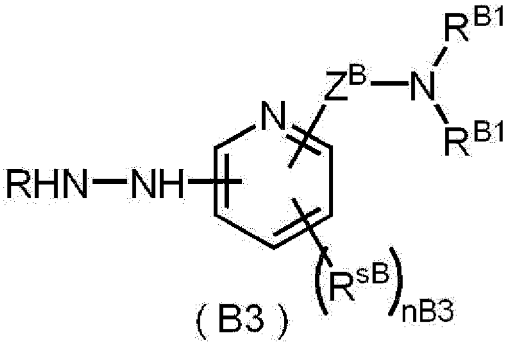

(B3)

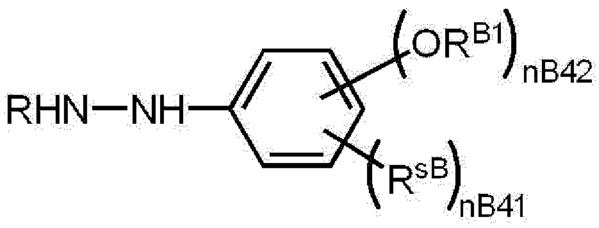

(B4)

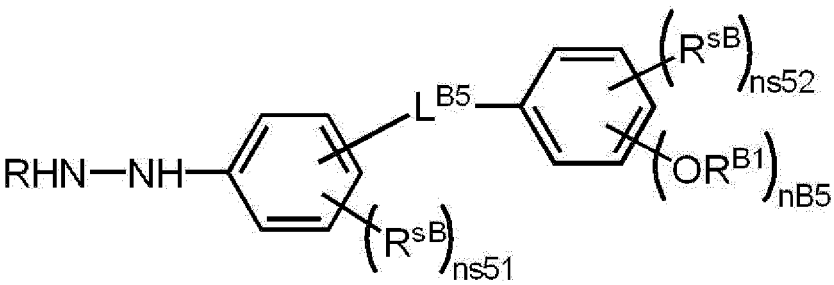

(B5)

In Formulae (B1) to (B5), $R^{B1}$'s each independently represent an aliphatic hydrocarbon group having 13 or more carbon atoms, $Z^B$ represents a carbonyl group or a sulfonyl group, $R^{sB}$'s each independently represent a substituent, nB1's each independently represent an integer of 0 to 4, nB2 represents an integer of 0 to 6, nB3 represents an integer of 0 to 3, nB41 represents an integer of 0 to 4, nB42 represents an integer of 1 to 5, nB5 represents an integer of 1 to 5, $L^{B5}$ represents a divalent linking group, R represents a hydrogen atom, an amino protective group, an amino acid residue, or a peptide residue, ns51 represents an integer of 0 to 4, ns52 represents an integer of 0 to 4, and nB5 + ns52 satisfies an integer of 1 to 5.

R in Formulae (B1) to (B5) has the same meanings as R in Formula (1), respectively, and the preferred aspect thereof is also the same.

From the viewpoint of yield in a case of peptide compound synthesis and availability of raw materials, $R^{B1}$'s in

Formulae (B1) to (B5) are each independently preferably an aliphatic hydrocarbon group having 16 to 40 carbon atoms, more preferably an aliphatic hydrocarbon group having 18 to 36 carbon atoms, still more preferably an alkyl group having 18 to 32 carbon atoms, and particularly preferably a linear alkyl group having 18 to 22 carbon atoms.

In addition, from the viewpoint of synthetic aptitude, $R^{B1}$'s in Formulae (B1) to (B5) are preferably all the same group.

From the viewpoint of solubility and crystallization property, nB5 in Formula (B5) is preferably an integer of 1 to 4, more preferably an integer of 2 to 4, and particularly preferably 2 or 3.

Preferred examples of $R^{sB}$ in Formulae (B1) to (B5) include the same substituents as the above-described substituent which may be included in the ring A.

nB1 in Formula (B1) is each independently preferably an integer of 0 to 2, more preferably 0 or 1, and particularly preferably 0.

nB2 in Formula (B2) is preferably an integer of 0 to 4, more preferably an integer of 0 to 2, and particularly preferably 0.

nB3 in Formula (B3) is preferably an integer of 0 to 2, more preferably 0 or 1, and particularly preferably 0.

nB41 in Formula (B4) is preferably 0 or 1, and more preferably 0.

nB42 in Formula (B4) is preferably an integer of 2 to 5, more preferably 2 to 4, and particularly preferably 2 or 3.

From the viewpoint of synthetic aptitude and crystallization property, the number of carbon atoms in $L^{B5}$ of Formula (B5) is preferably 1 to 30, more preferably 1 to 20, and particularly preferably 1 to 8.

From the viewpoint of synthetic aptitude and crystallization property, $L^{B5}$ in Formula (B5) is preferably an alkylene group or a group obtained by combining one or more of at least one bond selected from the group consisting of an ester bond, an amide bond, a sulfonamide bond, an ether bond, a urethane bond, and a urea bond with one or more alkylene groups,

more preferably a group obtained by combining one or more of at least one bond selected from the group consisting of an ester bond, an amide bond, a sulfonamide bond, an ether bond, a urethane bond, and a urea bond with one or more alkylene groups,
still more preferably a group obtained by combining one or more of at least one bond selected from the group consisting of an ester bond, an amide bond, and a sulfonamide bond with one or more alkylene groups, and
particularly preferably a group obtained by combining one or two of at least one bond selected from the group consisting of an ester bond, an amide bond, and a sulfonamide bond with one or two alkylene groups.

Furthermore, a substitution position of $-OR^{B1}$ in Formula (B5) on the benzene ring is not particularly limited, but is preferably 3rd to 5th position with respect to the substitution position (1st position) of $L^{B5}$.

ns51 in Formula (B5) is preferably an integer of 0 to 2, more preferably 0 or 1, and particularly preferably 0.
ns52 in Formula (B5) is preferably an integer of 0 to 2, more preferably 0 or 1, and particularly preferably 0.

The molecular weight of the hydrazine derivative represented by Formula (1) is not particularly limited, but from the viewpoint of deprotection rate, crystallization property, solubility in a solvent, by-product inhibitory property of diketopiperazine compound, yield in a case of peptide compound synthesis, and yield in a case of deprotection, it is preferably 340 to 3,000, more preferably 400 to 2,000, still more preferably 500 to 1,500, and particularly preferably 800 to 1,300. In addition, in a case where the molecular weight is 3,000 or less, the proportion of the hydrazine derivative represented by Formula (1) in the target product is appropriate and the proportion of a compound obtained by deprotecting the hydrazine derivative represented by Formula (1) is not reduced, so that productivity is excellent.

Preferred specific examples of the hydrazine derivative represented by Formula (1) include compounds shown below, but needless to say, the hydrazine derivative represented by Formula (1) is not limited thereto.

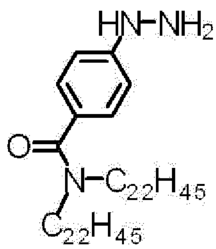 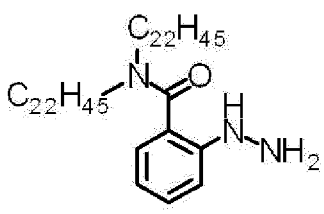 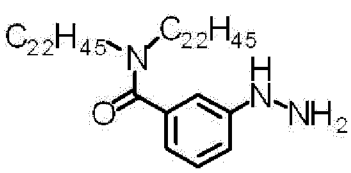 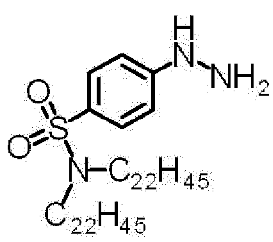

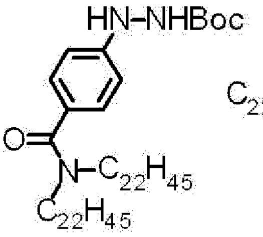

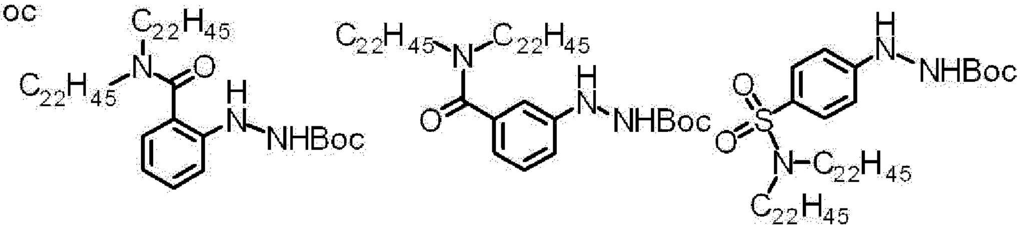

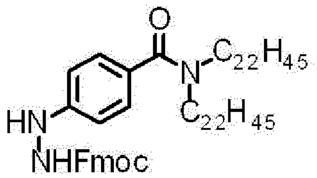

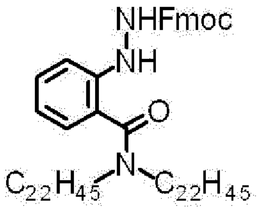

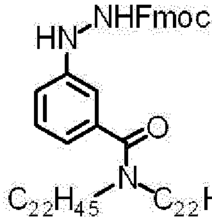

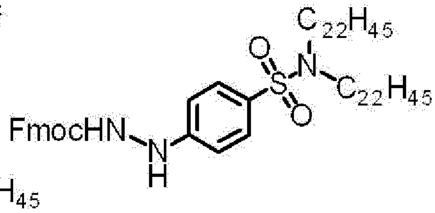

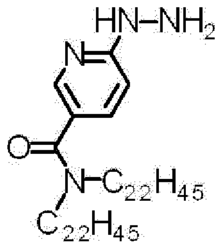

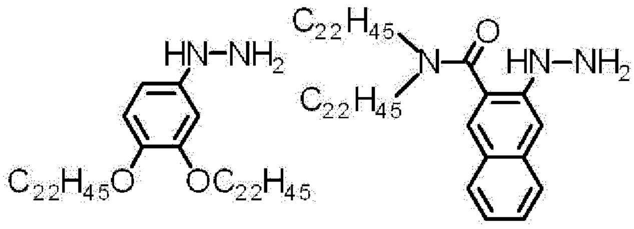

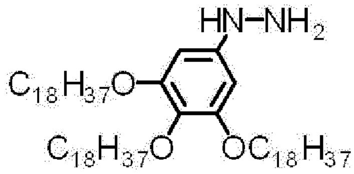

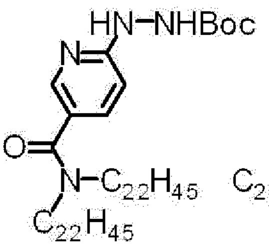

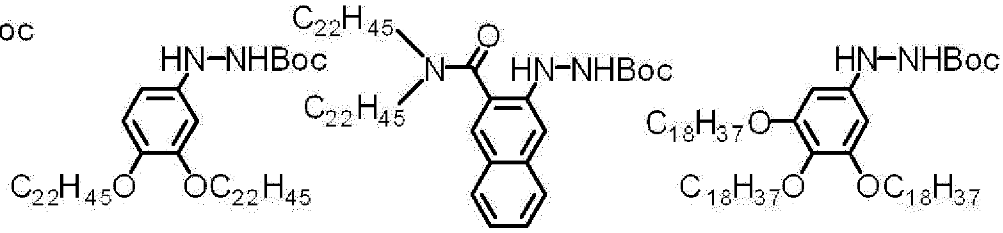

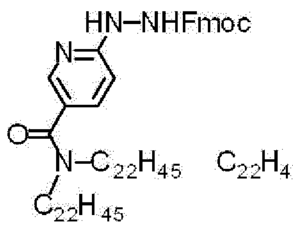

[0085]

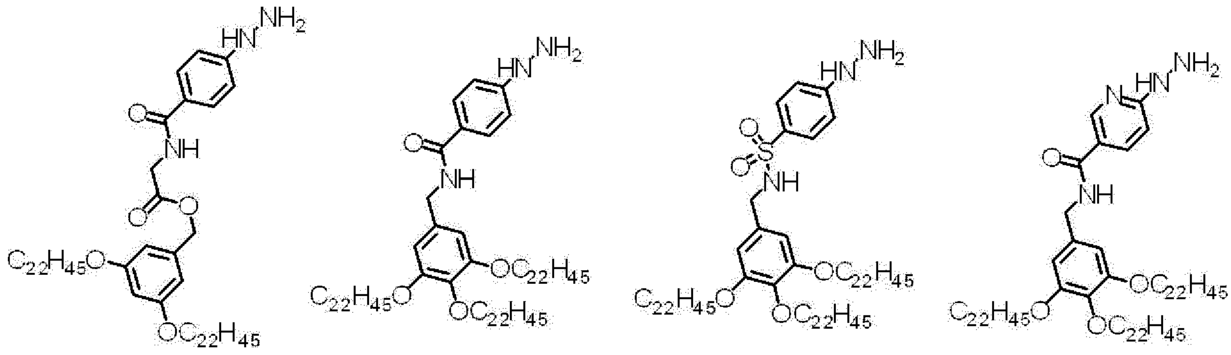

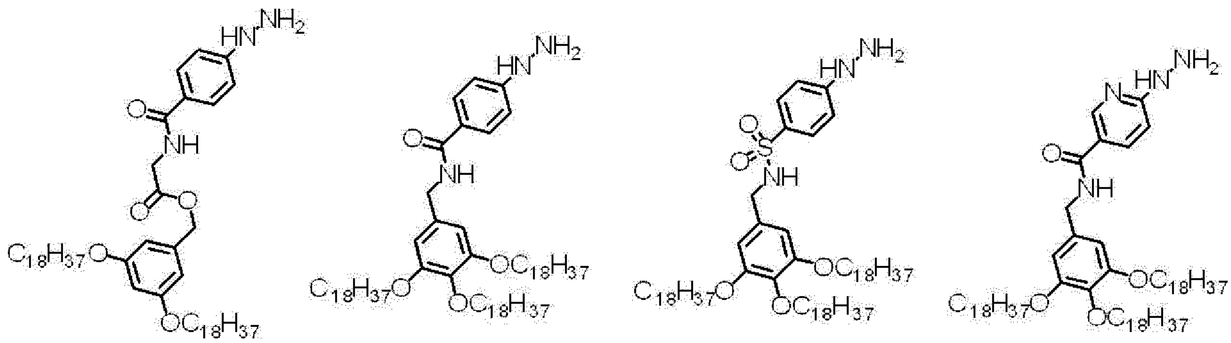

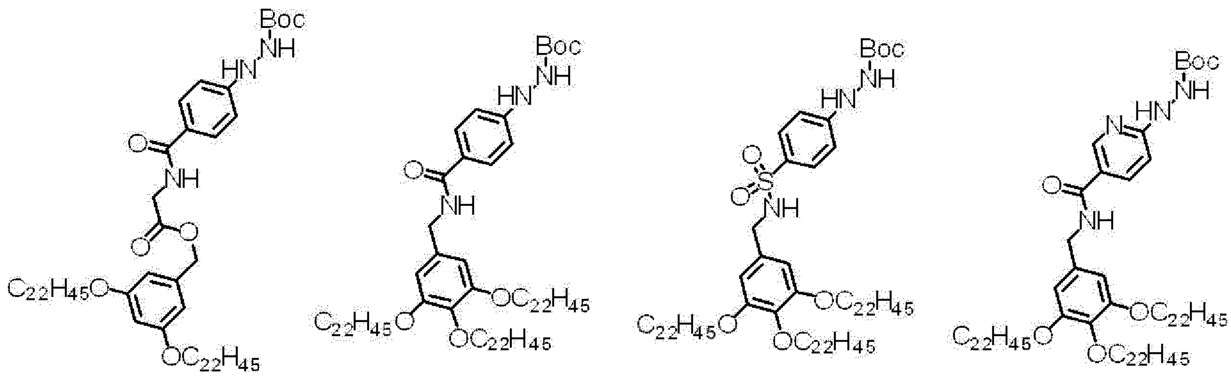

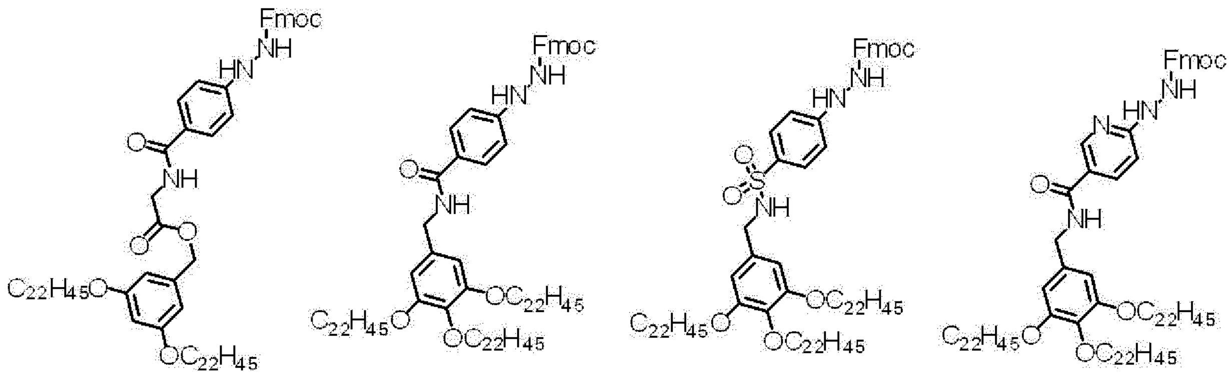

<Method for producing hydrazine derivative represented by Formula (1)>

A method for producing the hydrazine derivative represented by Formula (1) is not particularly limited, and the hydrazine derivative represented by Formula (1) can be produced by referring to a known method.

Unless otherwise specified, a raw material compound used for producing the hydrazine derivative represented by Formula (1) may be a commercially available compound, or may be produced by a known method or a method according to the known method. For example, by introducing a group having an aliphatic hydrocarbon group having 13

or more carbon atoms into an aromatic hydrazine derivative such as tert-butoxycarbonylhydrazinelbenzoic acid, the hydrazine derivative represented by Formula (1) can be suitably produced.

In addition, the produced hydrazine derivative represented by Formula (1) may be purified by a known purification method as necessary. For example, a method of isolating and purifying by recrystallization, column chromatography, or the like, a method of purifying by reprecipitation with a unit for changing the solution temperature, a unit for changing the solution composition, or the like, and the like can be performed.

In the method for producing a peptide compound according to the embodiment of the present disclosure, it is preferable that the step of using the hydrazine derivative represented by Formula (1) is a C-terminal protecting step of protecting a carboxy group of an amino acid compound or a peptide compound with the hydrazine derivative represented by Formula (1).

In addition, from the viewpoint of ease of synthesizing the peptide compound and yield, it is more preferable that the method for producing a peptide compound according to the embodiment of the present disclosure further includes, in addition to the above-described C-terminal protecting step of protecting a carboxy group of an amino acid compound or a peptide compound with the hydrazine derivative represented by Formula (1), an N-terminal deprotecting step of deprotecting an N-terminal of an N-terminal and C-terminal protected amino acid compound or an N-terminal and C-terminal protected peptide compound, which is obtained in the C-terminal protecting step, and a peptide chain extending step of condensing the N-terminal of a C-terminal protected amino acid compound or a C-terminal protected peptide compound, which is obtained in the N-terminal deprotecting step, with an N-terminal protected amino acid compound or an N-terminal protected peptide compound;

it is still more preferable that the method for producing a peptide compound according to the embodiment of the present disclosure further includes, in addition to the above steps, a precipitating step of precipitating the N-terminal and C-terminal protected peptide compound obtained in the peptide chain extending step or an extracting step of adding water to the N-terminal and C-terminal protected peptide compound obtained in the peptide chain extending step to extract and separate impurities into a water phase; and

it is particularly preferable that the method for producing a peptide compound according to the embodiment of the present disclosure further includes, one or more times in the following order after the precipitating step or the extracting step, a step of deprotecting the N-terminal of the obtained N-terminal and C-terminal protected peptide compound, a step of condensing the N-terminal of the obtained C-terminal protected peptide compound with an N-terminal protected amino acid compound or an N-terminal protected peptide compound, and a step of precipitating the obtained N-terminal and C-terminal protected peptide compound.

In addition, it is preferable that the method for producing a peptide compound according to the embodiment of the present disclosure further includes a C-terminal deprotecting step of deprotecting a C-terminal protective group.

Further, it is preferable that the method for producing a peptide compound according to the embodiment of the present disclosure further includes, before the above-described C-terminal protecting step, a dissolving step of dissolving the hydrazine derivative represented by Formula (1) in a solvent.

Furthermore, in a case where R in the hydrazine derivative represented by Formula (1) described above is an amino acid residue or a peptide residue, the hydrazine derivative represented by Formula (1) can be suitably used as a reaction substrate in the above-described N-terminal deprotecting step or the above-described peptide chain extending step.

Hereinafter, each step and the like described above will be described in detail.

<Dissolving step>

It is preferable that the method for producing a peptide compound according to the embodiment of the present disclosure includes, before the above-described C-terminal protecting step, a dissolving step of dissolving the hydrazine derivative represented by Formula (1) in a solvent.

As the solvent, a general organic solvent can be used for the reaction, but since excellent reactivity can be expected as solubility in the above-described solvent is higher, it is preferable to select a solvent having a high solubility of the hydrazine derivative represented by Formula (1). Specific examples thereof include halogenated hydrocarbons such as chloroform and dichloromethane; and nonpolar organic solvents such as 1,4-dioxane, tetrahydrofuran, cyclopentyl methyl ether, and 4-methyltetrahydropyran. Two or more of these solvents may be mixed and used in an appropriate ratio. In addition, as long as the hydrazine derivative represented by Formula (1) can be dissolved, in the above-described carbon halides or nonpolar organic solvents, aromatic hydrocarbons such as benzene, toluene, and xylene; nitriles such as acetonitrile and propionitrile; ketones such as acetone and 2-butanone; amides such as N,N-dimethylformamide and N-methylpyrrolidone; sulfoxides such as dimethyl sulfoxide; and esters such as isopropyl acetate and ethyl acetate may be mixed and used in an appropriate ratio.

In addition, a solvent described in Organic Process Research & Development, 2017, 21, 3, 365 to 369 may be used.

<Amino protective group deprotecting step on hydrazino group>

In the method for producing a peptide compound according to the embodiment of the present disclosure, in a case where a compound in which R in the hydrazine derivative represented by Formula (1) described above is an amino protective group is used, it is preferable to include an amino protective group deprotecting step on the hydrazino group of deprotecting the amino protective group of R in the hydrazine derivative represented by Formula (1).

In addition, the above-described amino protective group deprotecting step on the hydrazino group may be performed before or at the same time as the C-terminal protecting step described later.

Furthermore, the hydrazine derivative represented by Formula (1) that R in which the above-described amino protective group has been deprotected is a hydrogen atom may be used in the C-terminal protecting step described later while being isolated, or may be used as it is in the C-terminal protecting step described later without being isolated.

The deprotection condition is appropriately selected depending on the type of the above-described amino protective group, but a group which can be deprotected under conditions different from the removal of a protective group at the N terminal, which will be described later, is preferable. For example, in a case of the Fmoc group, the deprotection is performed by treating with a base, and in a case of the Boc group, the deprotection is performed by treating with an acid. The reaction is performed in a solvent which does not affect the reaction.

Examples of the base include secondary amines such as dimethylamine and diethylamine, and non-nucleophilic organic bases such as 1,8-diazabicyclo[5.4.0]-7-undecene (DBU), 1,4-diazabicyclo[2.2.2]octane (DABCO), and 1,5-diazabicyclo[4.3.0]-5-nonene (DBN).

As the solvent, the above-described solvent used in the above-described dissolving step can be suitably used.

<C-terminal protecting step>

It is preferable that the method for producing a peptide compound according to the embodiment of the present disclosure includes a C-terminal protecting step of protecting a carboxy group of an amino acid compound or a peptide compound with the hydrazine derivative represented by Formula (1).

The amino acid compound or peptide compound used in the above-described C-terminal protecting step is not particularly limited, and a known compound can be used. However, an N-terminal protected amino acid compound or an N-terminal protected peptide compound is preferable, and an Fmoc-protected amino acid compound or an Fmoc-protected peptide compound is more preferable.

In the method for producing a peptide compound according to the embodiment of the present disclosure, from the viewpoint of exerting the above-described effects more because the by-product of the diketopiperazine compound can be suppressed, it is preferable to use a peptide compound having at least one residue selected from the group consisting of a proline residue, a glycine residue, an N-alkylamino acid residue, peptoid, and a D-amino acid residue, which are peptide compounds in which the diketopiperazine compound is likely to be by-produced, it is more preferable to use a peptide compound having a proline residue or a glycine residue, and it is particularly preferable to use a peptide compound having a proline residue.

In addition, the position and number of the proline residue, glycine residue, N-alkylamino acid residue, peptoid, and D-amino acid residue in the above-described peptide compound are not particularly limited, but it is preferable that the amino acid residue at the C-terminal or the amino acid residue next to the C-terminal amino acid residue is at least a proline residue, a glycine residue, an N-alkylamino acid residue, a peptoid, or a D-amino acid residue, and it is more preferable that the C-terminal amino acid residue is at least a proline residue, a glycine residue, an N-methylamino acid residue, or a D-amino acid residue.

In addition, it is preferable that a hydroxy group, an amino group, a carbonyl group, an amide group, an imidazole group, an indole group, a guanidyl group, a mercapto group, or the like, which is a moiety other than the C-terminal in the amino acid compound or peptide compound used in the above-described C-terminal protecting step, is protected by the above-described known protective group.

The amount of the amino acid compound or peptide compound, which is used as a reaction substrate, to be used is preferably 1 molar equivalent to 10 molar equivalent, more preferably 1 molar equivalent to 5 molar equivalent, still more preferably 1 molar equivalent to 2 molar equivalent, and particularly preferably 1 molar equivalent to 1.5 molar equivalent with respect to 1 molar equivalent of the hydrazine derivative represented by Formula (1).

In addition, in the above-described C-terminal protecting step, it is preferable to add a condensing agent in the presence of a condensation additive (condensation activating agent) in a solvent which does not affect the reaction, or to react in an acid catalyst.

The amount of the condensation additive to be used is preferably 0.05 molar equivalent to 1.5 molar equivalent

with respect to 1 molar equivalent of the hydrazine derivative represented by Formula (1).

As the condensing agent, a condensing agent generally used in peptide synthesis can be used without limitation in the present disclosure. Examples thereof include 4-(4,6-dimethoxy-1,3,5-triazin-2-yl)-4-methylmorpholinium chloride (DMTMM), O-(benzotriazol-1-yl)-1,1,3,3-tetramethyluronium hexafluorophosphate (HBTU), O-(7-azabenzotriazol-1-yl)-1,1,3,3-tetramethyluronium hexafluorophosphate (HATU), O-(6-chlorobenzotriazol-1-yl)-1,1,3,3-tetramethyluronium hexafluorophosphate (HBTU(6-Cl)), O-(benzotriazol-1-yl)-1,1,3,3-tetramethyluronium tetrafluoroborate (TBTU), O-(6-chlorobenzotriazol-1-yl)-1,1,3,3-tetramethyluronium tetrafluoroborate (TCTU), (1-cyano-2-ethoxy-2-oxoethylidenaminooxy)dimethylamino-morpholino-carbenium hexafluorophosphate (COMU), dicyclohexylcarbodiimide (DCC), diisopropylcarbodiimide (DIC), 1-ethyl-3-(3-dimethylaminopropyl) carbodiimide (EDC), a hydrochloride salt (EDC/HCl) of 1-ethyl-3-(3-dimethylaminopropyl) carbodiimide, (7-azabenzotriazol-1-yloxy)tripyrrolidinophosphonium hexafluorophosphate (PyAOP), and (benzotriazol-1-yloxy)tripyrrolidinophosphonium hexafluorophosphate (PyBoP), but the condensing agent is not limited thereto.

Among these, DIC, EDC, EDC/HCl, DMT-MM, HBTU, HATU, COMU, or PyAOP is preferable.

The amount of the condensing agent to be used is preferably 1 molar equivalent to 10 molar equivalent and more preferably 1 molar equivalent to 5 molar equivalent with respect to 1 molar equivalent of the hydrazine derivative represented by Formula (1).

As the acid catalyst used in the condensation reaction, an acid catalyst generally used in peptide synthesis can be used without limitation, and examples thereof include trifluoroacetic acid (TFA), hydrochloric acid, trifluoroethanol (TFE), hexafluoroisopropanol (HFIP), acetic acid, methanesulfonic acid, trifluoromethanesulfonic acid, and p-toluenesulfonic acid.

Among these, trifluoroacetic acid is preferable.

The amount of the acid catalyst to be used is preferably more than 0 molar equivalent to 4.0 molar equivalent, more preferably 0.05 molar equivalent to 1.5 molar equivalent, and still more preferably 0.1 molar equivalent to 0.3 molar equivalent with respect to 1 molar equivalent of the hydrazine derivative represented by Formula (1).

In the above-described C-terminal protecting step, it is preferable to add a condensation activating agent in order to promote the reaction and suppress side reactions such as racemization.

The condensation activating agent in the present disclosure is a reagent which, in a case of coexisting with the condensing agent, leads an amino acid to a corresponding active ester, symmetric acid anhydride, or the like to facilitate the formation of a peptide bond (amide bond).

As the condensation activating agent, a condensation activating agent generally used in peptide synthesis can be used without limitation, and examples thereof include 4-dimethylaminopyridine, N-methylimidazole, boronic acid derivative, 1-hydroxybenzotriazole (HOBt), ethyl 1-hydroxytriazole-4-carboxylate (HOCt), 1-hydroxy-7-azabenzotriazole (HOAt), 3-hydroxy-1,2,3-benzotriazin-4(3H)-one (HOOBt), N-hydroxysuccinimide (HOSu), N-hydroxyphthalimide (HOPht), N-hydroxy-5-norbornene-2,3-dicarboxyimide (HONb), pentafluorophenol, and ethyl(hydroxyimino)cyanoacetylate (Oxyma). Among these, 4-dimethylaminopyridine, HOBt, HOCt, HOAt, HOOBt, HOSu, HONb, or Oxyma is preferable.

The amount of the activating agent to be used is preferably more than 0 molar equivalent to 4.0 molar equivalent and more preferably 0.1 molar equivalent to 1.5 molar equivalent with respect to the amino acid compound or peptide compound.

As the base, a base generally used in peptide synthesis can be used without limitation, and examples thereof include a tertiary amine such as diisopropylethylamine.

As the solvent, the above-described solvent used in the above-described dissolving step can be suitably used.

The reaction temperature is not particularly limited, but is preferably -10°C to 80°C and more preferably 0°C to 40°C. The reaction time is not particularly limited, but is preferably 1 hour to 30 hours.

To confirm the progress of the reaction, a method same as that of a general liquid phase organic synthesis reaction can be applied. That is, the reaction can be traced using thin-layer silica gel chromatography, high performance liquid chromatography, NMR, or the like.

In addition, the N-terminal and C-terminal protected amino acid compound or N-terminal and C-terminal protected peptide compound obtained by the above-described C-terminal protecting step may be purified.

For example, in order to isolate the product obtained after dissolving the obtained N-terminal and C-terminal protected amino acid compound or N-terminal and C-terminal protected peptide compound in a solvent and performing a desired organic synthesis reaction, it is preferable to perform a method of changing the solvent to a solvent in which the N-terminal and C-terminal protected amino acid compound or N-terminal and C-terminal protected peptide compound is dissolved (for example, change of solvent composition or change of solvent type) and reprecipitating the resultant.

Specifically, for example, the reaction is performed under conditions such that the N-terminal and C-terminal protected amino acid compound or N-terminal and C-terminal protected peptide compound is dissolved, and after the reaction, the solvent is distilled off and then replaced, or after the reaction, by adding a polar solvent to the reaction system without distilling off the solvent, aggregates are precipitated and impurities are eliminated. As the solvent for

replacement, polar organic solvents such as methanol, acetonitrile, and water are used alone or in combination. That is, the reaction is performed under conditions such that the N-terminal and C-terminal protected amino acid compound or N-terminal and C-terminal protected peptide compound is dissolved, and in the solvent replacement after the reaction, for example, a halogenated solvent, THF, or the like is used for dissolution, and a polar organic solvent such as methanol, acetonitrile, and water, or a mixed solvent thereof is used for precipitation.

<N-terminal deprotecting step>

It is preferable that the method for producing a peptide compound according to the embodiment of the present disclosure includes an N-terminal deprotecting step of deprotecting the N-terminal of the N-terminal and C-terminal protected amino acid compound or N-terminal and C-terminal protected peptide compound, which is obtained in the C-terminal protecting step.

As the N-terminal protective group, the above-described protective group for an amino group, which is generally used in technical fields such as peptide chemistry, can be used, but in the present disclosure, a Boc group, a Cbz group, or an Fmoc group is suitably used.

The deprotection condition is appropriately selected depending on the type of the temporary protective group, but a group which can be deprotected under conditions different from the removal of the protective group derived from the hydrazine derivative represented by Formula (1) (the step of deprotecting the amino protective group on the hydrazino group) is preferable. For example, in a case of the Fmoc group, the deprotection is performed by treating with a base, and in a case of the Boc group, the deprotection is performed by treating with an acid. The reaction is performed in a solvent which does not affect the reaction.

As the base, the above-described base in the amino protective group deprotecting step on the hydrazino group can be suitably used.

As the solvent, the above-described solvent used in the above-described dissolving step can be suitably used.

<Peptide chain extending step>

It is preferable that the method for producing a peptide compound according to the embodiment of the present disclosure includes a peptide chain extending step of condensing the N-terminal of a C-terminal protected amino acid compound or a C-terminal protected peptide compound, which is obtained in the N-terminal deprotecting step, with an N-terminal protected amino acid compound or an N-terminal protected peptide compound.

The above-described peptide chain extending step is suitably performed under peptide synthesis conditions generally used in the field of peptide chemistry, in which the above-described condensing agent, condensation additive, and the like are used.

The N-terminal protected amino acid compound or N-terminal protected peptide compound is not particularly limited, and a desired compound can be used. However, an Fmoc-protected amino acid compound or an Fmoc-protected peptide compound can be suitably used.

In addition, it is preferable that a hydroxy group, an amino group, a carbonyl group, an amide group, an imidazole group, an indole group, a guanidyl group, a mercapto group, or the like, which is a moiety other than the C-terminal in the N-terminal protected amino acid compound or N-terminal protected peptide compound, is protected by a known protective group such as a protective group described later.

<Precipitating step>

It is preferable that the method for producing a peptide compound according to the embodiment of the present disclosure further includes a precipitating step of precipitating the N-terminal and C-terminal protected peptide compound obtained in the peptide chain extending step.

The above-described precipitating step can be performed in the same manner as the precipitation method in the purification which may be performed after the above-described C-terminal protecting step.

<Extracting step>

It is preferable that the method for producing a peptide compound according to the embodiment of the present disclosure further includes an extracting step of adding water to the N-terminal and C-terminal protected peptide compound obtained in the peptide chain extending step to extract and separate impurities into a water phase.

In the above-described extracting step, for example, water is added to the N-terminal and C-terminal protected peptide compound obtained in the above-described peptide chain extending step or to a compound in which an organic solvent is added to the N-terminal and C-terminal protected peptide compound, a liquid separation operation was performed, impurities are extracted into the water phase, the water phase is separated from an oil phase or the N-terminal and C-terminal protected peptide compound, and as necessary, the organic solvent is removed from the oil phase to obtain a purified N-terminal and C-terminal protected peptide compound.

As the organic solvent, the above-described organic solvent the dissolving step can be used.

Extraction conditions and extraction operations in the above-described extracting step are not particularly limited, and the extracting step can be performed using known extraction conditions and extraction operations.

<Chain extension>

It is preferable that the method for producing a peptide compound according to the embodiment of the present disclosure further includes, one or more times in the following order after the precipitating step, a step of deprotecting the N-terminal of the obtained N-terminal and C-terminal protected peptide compound, a step of condensing the N-terminal of the obtained C-terminal protected peptide compound with an N-terminal protected amino acid compound or an N-terminal protected peptide compound, and a step of precipitating the obtained N-terminal and C-terminal protected peptide compound.

By repeating the above-described three steps, the chain extension of the obtained peptide compound can be easily performed.

Each step in the above-described three steps can be performed in the same manner as each corresponding step described above.

<C-terminal deprotecting step>

It is preferable that the method for producing a peptide compound according to the embodiment of the present disclosure further includes a C-terminal deprotecting step of deprotecting a C-terminal protective group.

In the above-described C-terminal deprotecting step, by removing the C-terminal protective group formed by the hydrazine derivative represented by Formula (1) in the C-terminal protected peptide compound having a desired number of amino acid residues, a peptide compound, which is the final target product, can be obtained.

Preferred examples of a method of removing the C-terminal protective group include a deprotecting method using an oxidizing agent.

As the oxidizing agent, any of an inorganic oxidizing agent or an organic oxidizing agent can be used, and a known oxidizing agent can be used.

As the inorganic oxidizing agent, a metal hydrochloric acid agent is preferable, a copper hydrochloric acid agent is more preferable, and a divalent copper salt is particularly preferable.

Examples of the divalent copper salt include copper bromide, copper chloride, copper fluoride, copper hydroxide, copper phosphate, copper acetate, copper dimethyldithiocarbamate, copper methoxide, copper ethoxide, copper isopropoxide, copper ethylacetate, copper 2-ethylhexanoate, copper gluconate, copper hexafluoroacetylacetonate, copper isobutyrate, copper phthalate, copper trifoloacetylacetonate, and copper trifluoromethanesulfonate.

As the organic oxidizing agent, quinones, hypervalent iodine, an organic peroxide, or a compound having a nitrogen-halogen bond is preferable.

Examples of an N-halogenated nitrogen-containing compound include imide compounds such as N-chlorosuccinimide, N-bromosuccinimide, N-iodosuccinimide, N-chlorophthalimide, N-bromophthalimide, N-iodophthalimide, N-chloroacetimide, N-bromoacetimide, and N-iodoacetimide; hydantoin compounds such as 1,3-dichloro-2,5-dimethylhydantoin, 1,3-dibromo-2,5-dimethylhydantoin, and 1,3-diiodo-2,5-dimethylhydantoin; isocyanuric acid compounds such as 1,3,5-trichloroisocyanuric acid, 1,3,5-tribromoisocyanuric acid, and 1,3,5-triiodoisocyanuric acid.

Among these, as the oxidizing agent, a divalent copper salt, quinones, or an N-halogenated nitrogen-containing compound is preferable, a divalent copper halide, a divalent copper carboxylic acid salt, benzoquinone, or an N-halogenated imide compound is more preferable, and a divalent copper acetate, 1,4-benzoquinone, or N-bromosuccinimide is particularly preferable.

As the solvent, the above-described solvent used in the above-described dissolving step, a polar solvent such as water, an alcohol compound, and acetonitrile, or a mixed solvent thereof can be suitably used.

The reaction temperature is not particularly limited, but is preferably -10°C to 80°C and more preferably 0°C to 40°C. The reaction time is not particularly limited, but is preferably 0.1 hours to 30 hours.

The peptide compound, which is the final target product obtained by the method for producing a peptide compound according to the embodiment of the present disclosure, can be isolated and purified according to a method commonly used in peptide chemistry. For example, the peptide compound, which is the final target product, can be isolated and purified by extraction and washing the reaction mixture, crystallization, chromatography, and the like.

The type of peptide produced by the method for producing a peptide compound according to the embodiment of the present disclosure is not particularly limited, but it is preferable that the number of amino acid residues of the

peptide compound is, for example, approximately several tens or less. Same as existing or unknown synthetic or native peptides, the peptide obtained by the method for producing a peptide compound according to the embodiment of the present disclosure can be used in various fields such as pharmaceuticals, foods, cosmetics, electronic materials, biosensors, and the like, but the use of the peptide is not limited thereto.

In the method for producing a peptide compound according to the embodiment of the present disclosure, the precipitating step can be appropriately omitted as long as it does not affect the reaction in the next step.

In a case where the amino acid compound or peptide compound used in the method for producing a peptide compound according to the embodiment of the present disclosure has a hydroxy group, an amino group, a carboxy group, a carbonyl group, a guanidyl group, a mercapto group, or the like, a protective group generally used in peptide chemistry or the like may be introduced into these groups, and the target compound can be obtained by removing the protective group as necessary after the reaction.

Examples of these protective groups include those described above.

In addition, the method of removing these protective groups may be performed according to a known method in this field, for example, a method described in Greene's Protective Groups in Organic Synthesis (2014). For example, a method using acid, base, ultraviolet light, hydrazine, phenylhydrazine, sodium N-methyldithiocarbamate, tetrabutylammonium fluoride, palladium acetate, trialkylsilyl halide (for example, trimethylsilyl iodide and trimethylsilyl bromide), or the like, a reduction method, and the like are used.

(reagent for forming a protective group)

The reagent for forming a protective group according to an embodiment of the present disclosure includes a hydrazine derivative represented by Formula (1a).

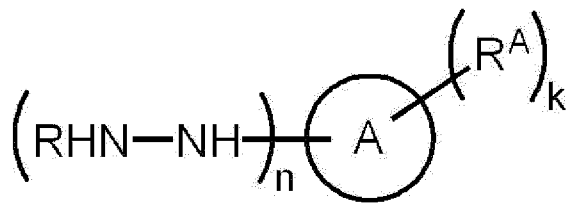

(1a)

In Formula (1a),

a ring A represents an aromatic hydrocarbon ring or an aromatic heterocyclic ring,
R represents a hydrogen atom or an amino protective group,
k represents an integer of 1 to 5,
n represents 1 or 2,
$R^A$'s each independently represent an aliphatic hydrocarbon group or an organic group having an aliphatic hydrocarbon group, and
at least one aliphatic hydrocarbon group in k pieces of $R^A$'s has 13 or more carbon atoms.

The reagent for forming a protective group according to the embodiment of the present disclosure is preferably a reagent for forming a protective group of a carboxy group, and more preferably a C-terminal reagent for forming a protective group of an amino acid compound or a peptide compound.

Apreferred aspect of the hydrazine derivative represented by Formula (1a) in the reagent for forming a protective group according to the embodiment of the present disclosure is the same as the preferred aspect of the hydrazine derivative represented by Formula (1) in the method for producing a peptide compound according to the embodiment of the present disclosure described above, except that R is a hydrogen atom or an amino protective group.

In addition, a preferred aspect of R of the hydrazine derivative represented by Formula (1a) in the reagent for forming a protective group according to the embodiment of the present disclosure is the same as the preferred aspect of R of the hydrazine derivative represented by Formula (1) in the method for producing a peptide compound according to the embodiment of the present disclosure described above, in which the amino acid residue and the peptide residue are excluded.

The reagent for forming a protective group according to the embodiment of the present disclosure may be a solid reagent or a liquid reagent.

The content of the hydrazine derivative represented by Formula (1a) in the reagent for forming a protective group according to the embodiment of the present disclosure is not particularly limited, but is preferably 0.1% by mass to 100% by mass, more preferably 1% by mass to 100% by mass, and still more preferably 3% by mass to 100% by

mass with respect to the total mass of the reagent for forming a protective group.

The reagent for forming a protective group according to the embodiment of the present disclosure may include a component other than the hydrazine derivative represented by Formula (1a).

As the other components, a known component can be included. Examples thereof include water, an organic solvent, an antioxidant, and a pH adjuster.

(Hydrazine derivative represented by Formula (1b))

The compound according to the present disclosure is a hydrazine derivative represented by Formula (1b).

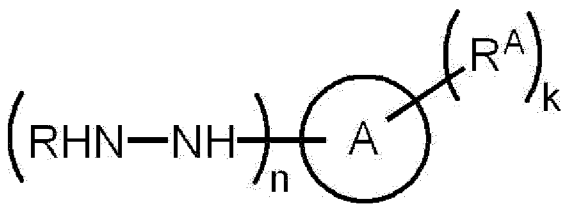

(1b)

In Formula (1b),

a ring A represents an aromatic hydrocarbon ring or an aromatic heterocyclic ring,
R represents a hydrogen atom or an amino protective group,
k represents an integer of 1 to 5,
n represents 1 or 2,
$R^A$'s each independently represent an aliphatic hydrocarbon group or an organic group having an aliphatic hydrocarbon group, and
at least two aliphatic hydrocarbon groups in k pieces of $R^A$'s have 13 or more carbon atoms.

The hydrazine derivative represented by Formula (1b), which is the compound according to the present disclosure, is a novel compound and can be suitably used for producing a peptide compound. Among these, the hydrazine derivative represented by Formula (1b) can be suitably used as a reagent for forming a protective group, more suitably used as a reagent for forming a protective group of a carboxy group, and particularly suitably used as a C-terminal reagent for forming a protective group of an amino acid compound or a peptide compound.

The hydrazine derivative represented by Formula (1b) in the compound according to the present disclosure is the same as the hydrazine derivative represented by Formula (1) in the method for producing a peptide compound according to the embodiment of the present disclosure, except that R is a hydrogen atom or an amino protective group and at least two aliphatic hydrocarbon groups in k pieces of $R^A$'s have 13 or more carbon atoms, and the preferred aspects thereof are also the same.

In addition, a preferred aspect of R in the compound according to the present disclosure is the same as the preferred aspect of R in the hydrazine derivative represented by Formula (1) according to the embodiment of the present disclosure described above, in which the amino acid residue and the peptide residue are excluded.

In addition, the above-described hydrazine derivative represented by Formula (1b) can be synthesized in the same manner as in the hydrazine derivative represented by Formula (1) described above.

## Examples

Hereinafter, the embodiments of the present invention will be more specifically described with reference to Examples. The materials, amounts to be used, proportions, treatment contents, treatment procedures, and the like shown in Examples can be appropriately modified as long as the modifications do not depart from the spirit of the embodiments of the present invention. Therefore, the scope of the embodiments of the present invention is not limited to the following specific examples. In addition, "parts" and "%" are on a mass basis unless otherwise specified.

Unless otherwise specified, purification by silica gel column chromatography was performed using an automatic purification device ISOLERA (manufactured by Biotage Ltd.) or a medium pressure liquid chromatograph YFLC-Wprep 2XY.N (manufactured by YAMAZEN).

Unless otherwise specified, purification by high performance liquid chromatography (HPLC) was carried out by 1260 Infinity II LC System manufactured by Agilent Technologies, Inc. (column: PREP-C18 (50 mm x 21.2 mm) manufactured by Agilent Technologies, Inc., gradient conditions: $H_2O$ (0.1 %$HCO_2H$)/acetonitrile (0.1 %$HCO_2H$) = 90/10 → 30/70, flow rate: 20.0 ml/min).

Unless otherwise specified, SNAPKP-SII Cartridge (manufactured by Biotage Ltd.) or a high flash column W001, W002, W003, W004, or W005 (manufactured by YAMAZEN) was used as a carrier in silica gel column chromatography.

MS spectrum was measured using ACQUITY SQD LC/MS System (manufactured by Waters Corporation) or LC-MS2020 (manufactured by Shimadzu Corporation) by an ionization method; electrospray ionization (ESI) method.

NMR spectrum was measured using Bruker AV300 (manufactured by Bruker, 300 MHz) or Bruker AV400 (manufactured by Bruker, 400 MHz), using tetramethylsilane as an internal reference, and all δ values were shown in ppm.

<Synthesis of 3,5-bis(docosyloxy)benzyl=(((9H-fluoren-9-yl)methoxy)carbonyl) glycinate>

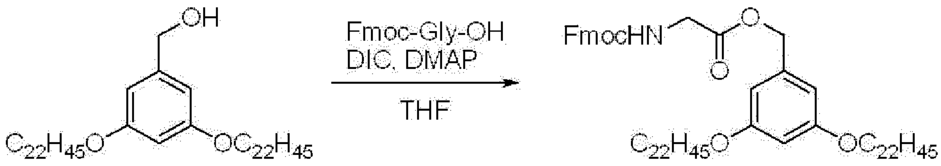


N-[(9H-fluoren-9-ylmethoxy)carbonyl]glycine (Fmoc-Gly-OH, 353 mg, 1.188 mmol), N,N'-diisopropylcarbodiimide (DIC, 186 μL, 1.188 mmol), and N,N-dimethyl-4-aminopyridine (DMAP, 19.4 mg, 0.159 mmol) were added to a tetrahydrofuran (THF) solution (15.9 mL) of 3,5-bis(docosyloxy)phenyl)methanol (600 mg, 0.792 mmol), and the mixture was stirred at room temperature (25°C; the same applies hereinafter) for 1 hour. The solid precipitated by adding acetonitrile was collected by filtration and dried under reduced pressure to obtain 3,5-bis(docosyloxy)benzyl=(((9H-fluoren-9-yl)methoxy)carbonyl) glycinate (741 mg, 0.715 mmol).

$^1$H-NMR ($CDCl_3$): δ: 7.76 (2H, d, J = 7.9 Hz), 7.60 (2H, d, J = 7.9 Hz), 7.46 to 7.30 (4H, m), 6.47 (2H, d, J = 2.0 Hz), 6.40 (1H, s), 5.11 (2H, s), 4.41 (2H, d, J = 6.6 Hz), 4.29 to 4.18 (1H, m), 4.06 (2H, d, J = 5.3 Hz), 3.96 to 3.85 (4H, m), 1.83 to 1.19 (80H, m), 0.88 (6H, t, J = 6.6 Hz)

<Synthesis of tert-butyl=2-(4-((2-(3,5-bis(docosyloxy)benzyloxy)-2-oxoethyl)carbamoyl)phenyl)hydrazine-1-carboxylate>

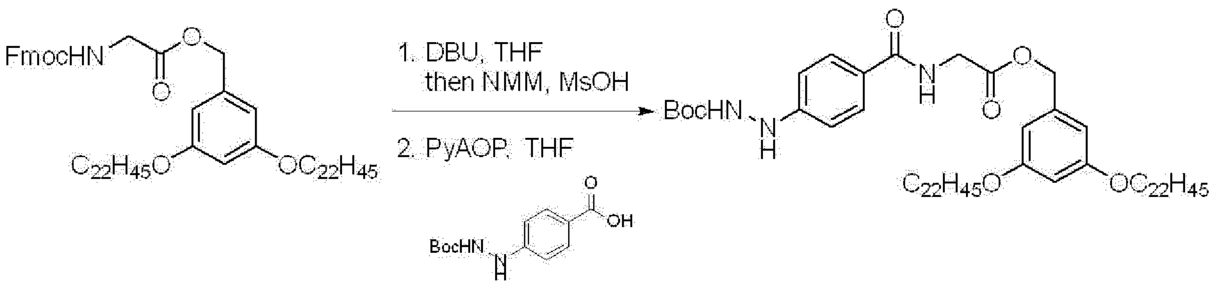


Diazabicycloundecene (DBU, 215 μL, 1.43 mmol) was added to a tetrahydrofuran solution (14.3 mL) of 3,5-bis(docosyloxy)benzyl=(((9H-fluoren-9-yl)methoxy)carbonyl) glycinate (741 mg, 0.715 mmol), and the mixture was stirred at room temperature for 1 hour. N-methylmorpholine (NMM, 158 μL, 1.43 mmol) and methanesulfonic acid (MsOH, 96.1 μL, 1.47 mmol) were added thereto, and the mixture was stirred at room temperature for 10 minutes. 4-(2-(tert-butoxycarbonyl)hydrazinyl) benzoic acid (225 mg, 0.894 mmol) and (7-azabenzotriazol-1-yloxy)tripyrrolidinophosphonium hexafluorophosphate (PyAOP, 466 mg, 0.894 mmol) were added thereto, and the mixture was stirred at room temperature for 2 hours and 30 minutes. The solid precipitated by adding acetonitrile was collected by filtration and dried under reduced pressure to obtain tert-butyl=2-(4-((2-(3,5-bis(docosyloxy)benzyloxy)-2-oxoethyl)carbamoyl)phenyl)hydrazine-1-carboxylate (356 mg, 0.340 mmol).

$^1$H-NMR ($CDCl_3$): δ: 7.71 (2H, d, J = 8.6 Hz), 6.82 (2H, d, J = 8.6 Hz), 6.63 to 6.33 (5H, m), 6.04 to 5.94 (1H, m), 5.13 (2H, s), 4.27 (2H, t, J = 4.6 Hz), 3.92 (4H, t, J = 6.6 Hz), 1.86 to 1.19 (89H, m), 0.88 (6H, t, J = 6.6 Hz)

(Example 1)

<Synthesis of (9H-fluoren-9-yl)methyl=(S)-2-(2-(4-((2((3,5-bis(docosyloxy)benzyl)oxy)-2-oxoethyl)carbamoyl)phenyl)phenyl)hydrazine)-1-carbonyl)pyrrolidine-1-carboxylate>

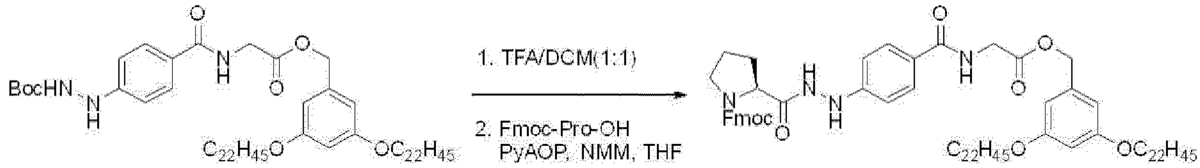


Trifluoroacetic acid (TFA, 6.6 mL) was added to a dichloromethane solution (DCM, 6.6 mL) of tert-butyl=2-(4-((2-(3,5-bis(docosyloxy)benzyloxy)-2-oxoethyl)carbamoyl)phenyl)hydrazine-1-carboxylate (356 mg, 0.340 mmol), and the mixture was stirred at room temperature for 1 hour and 30 minutes. The solvent was distilled off under reduced pressure, and tetrahydrofuran (6.8 mL) was added to the obtained white solid. N-[(9H-fluoren-9-ylmethoxy)carbonyl]-L-proline (Fmoc-Pro-OH, 172 mg, 0.509 mmol), (7-azabenzotriazol-1-yloxy)tripyrrolidinophosphonium hexafluorophosphate (266 mg, 0.509 mmol), and N-methylmorpholine (337 μL, 3.06 mmol) were added to the mixture, and the mixture was stirred at room temperature for 30 minutes. The solid precipitated by adding acetonitrile was collected by filtration to obtain (9H-fluoren-9-yl)methyl=(S)-2-(2-(4-((2((3,5-bis(docosyloxy)benzyl)oxy)-2-oxoethyl)carbamoyl)phenylhydrazine)-1-carbonyl)pyrrolidine-1-carboxylate (282 mg, 0.222 mmol).

$$\text{ESI-MS(-)} = 1{,}266.00$$

No residual raw material was observed during condensation.

<Synthesis of 3,5-bis(docosyloxy)benzyl=(4-(2-((((9H-fluoren-9-yl)methoxy)carbonyl)glycyl-L-prolyl)hydrazinyl)benzoyl) glycinate>

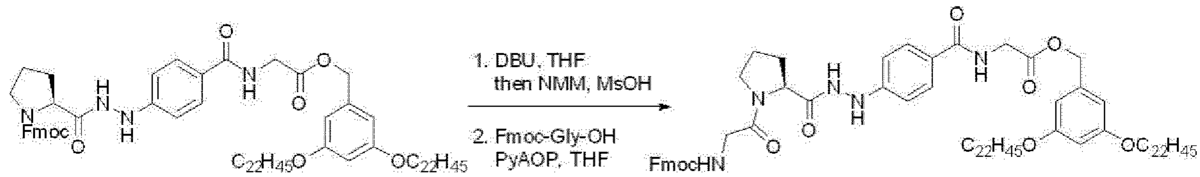


Diazabicycloundecene (101 μL, 0.674 mmol) was added to a tetrahydrofuran solution (7.4 mL) of (9H-fluoren-9-yl)methyl=(S)-2-(2-(4-((2((3,5-bis(docosyloxy)benzyl)oxy)-2-oxoethyl)carbamoyl)phenylhydrazine)-1-carbonyl)pyrrolidine-1-carboxylate (280 mg, 0.221 mmol), and the mixture was stirred at room temperature for 30 minutes. N-methylmorpholine (48.7 μL, 0.442 mmol) and methanesulfonic acid (44.2 μL, 0.674 mmol) were added thereto, and the mixture was stirred at room temperature for 10 minutes. N-[(9H-fluoren-9-ylmethoxy)carbonyl]-glycine (125 mg, 0.420 mmol) and cyano-2-ethoxy-2-oxoethylidenaminooxy)dimethylamino-morpholino-carbenium hexafluorophosphate (180 mg, 0.42 mmol) were added to the mixture, and the mixture was stirred at room temperature for 30 minutes. The solid precipitated by adding acetonitrile was collected by filtration to obtain 3,5-bis(docosyloxy)benzyl=(4-(2-((((9H-fluoren-9-yl)methoxy)carbonyl)glycyl-L-prolyl)hydrazinyl)benzoyl) glycinate (293 mg, 0.220 mmol).

ESI-MS(-) = 1,322.85

<Synthesis of 3,5-bis(docosyloxy)benzyl(4-(2-((tert-butoxycarbonyl)-L-phenylalanylglycyl-L-prolyl)hydrazinyl)benzoyl) glycinate>

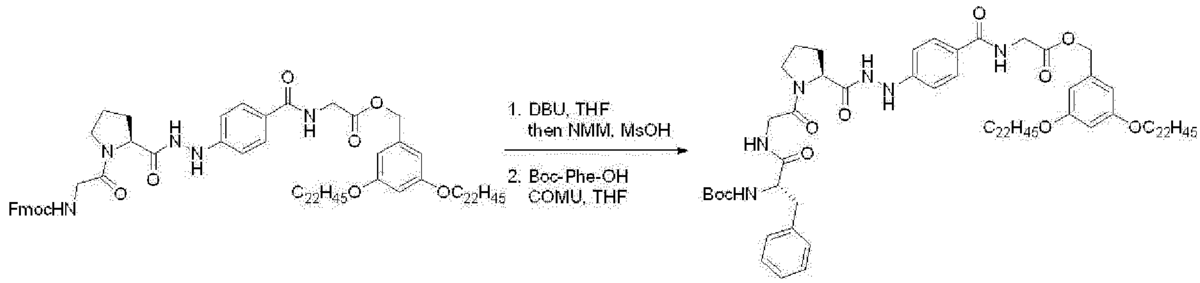


Diazabicycloundecene (65.8 μL, 0.438 mmol) was added to a tetrahydrofuran solution (5.0 mL) of 3,5-bis(docosyloxy)benzyl=(4-(2-((((9H-fluoren-9-yl)methoxy)carbonyl)glycyl-L-prolyl)hydrazinyl)benzoyl) glycinate (290 mg, 0.219 mmol), and the mixture was stirred at room temperature for 40 minutes. N-methylmorpholine (48.3 μL, 0.438 mmol) and methanesulfonic acid (29.4 μL, 0.449 mmol) were added thereto, and the mixture was stirred at room temperature for 10 minutes. N-(tert-butoxycarbonyl)-L-phenylalanine (Boc-Phe-OH, 72.6 mg, 0.274 mmol) and cyano-2-ethoxy-2-oxoethylidenaminooxy)dimethylamino-morpholino-carbenium hexafluorophosphate (COMU, 117 mg, 0.274 mmol) were added to the mixture, and the mixture was stirred at room temperature for 50 minutes. The solid precipitated by adding acetonitrile was collected by filtration to obtain 3,5-bis(docosyloxy)benzyl=(4-(2-((tert-butoxycarbonyl)-L-phenylalanylglycyl-L-prolyl)hydrazinyl)benzoyl) glycinate (216 mg, 0.160 mmol).

Formation of the diketopiperazine compound during the DBU treatment was not confirmed.

$$\text{ESI-MS}(-) = 1{,}347.85$$

<Synthesis (deprotection reaction) of tert-butyl=((S)-1-((2-((S)-2-(benzylcarbamoyl)pyrrolidin-1-yl)-2-oxoethyl)amino)-1-oxo-3-phenylpropan-2-yl) carbamate>

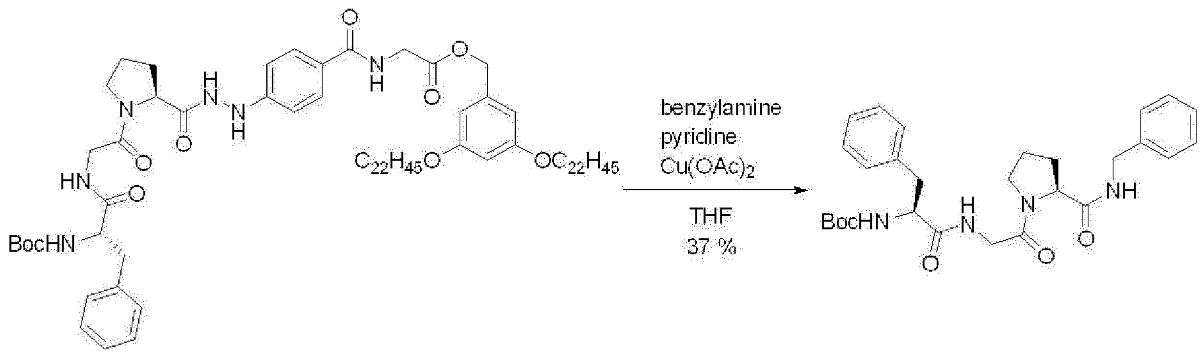


Benzylamine (3.30 μL), pyridine (15 μL, 0.185 mmol), and copper acetate monohydrate ($Cu(OAc)_2$, 0.37 mg, 0.00185 mmol) were added to a tetrahydrofuran solution (740 μL) of 3,5-bis(docosyloxy)benzyl=(4-(2-((tert-butoxycarbonyl)-L-phenylalanylglycyl-L-prolyl)hydrazinyl)benzoyl) glycinate (25.0 mg, 0.0185 mmol), and the mixture was stirred at room temperature for 2 hours. The solid precipitated by adding methanol was removed by centrifugation to obtain a supernatant including the target peptide compound. The solvent was distilled off under reduced pressure, and then pretreatment with a silica pad and purification by HPLC were performed to obtain (tert-butoxycarbonyl)-L-phenylalanylglycyl-L-proline (3.57 mg, 0.00683 mmol).

$$\text{ESI-MS}(+) = 509.3$$

(Example 2)

<Synthesis of (tert-butoxycarbonyl)-L-phenylalanylglycyl-L-proline>

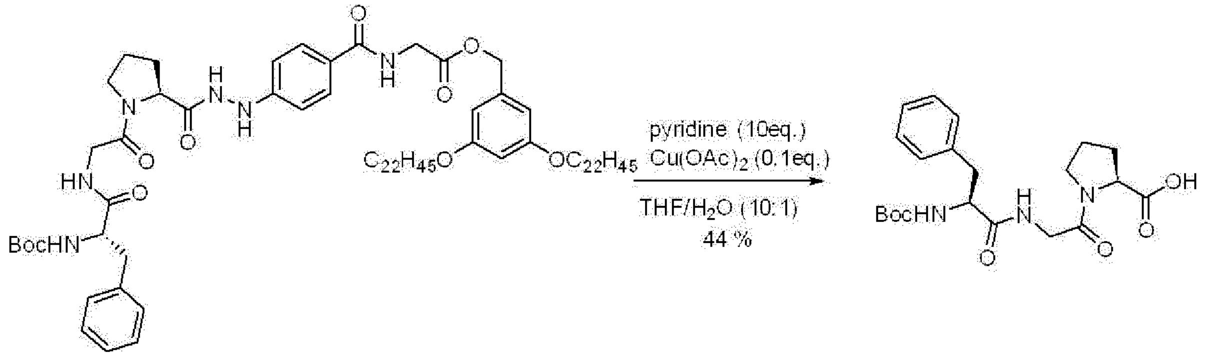


Water (74 μL), pyridine (15 μL, 0.185 mmol), and copper acetate monohydrate (0.37 mg, 0.00185 mmol) were added to a tetrahydrofuran solution (740 μL) of 3,5-bis(docosyloxy)benzyl=(4-(2-((tert-butoxycarbonyl)-L-phenylalanylglycyl-L-prolyl)hydrazinyl)benzoyl) glycinate (25.0 mg, 0.0185 mmol), and the mixture was stirred at room temperature for 2 hours. The solid precipitated by adding methanol was removed by centrifugation to obtain a supernatant including the target peptide compound. The solvent was distilled off under reduced pressure, and then pretreatment with a silica pad and purification by HPLC were performed to obtain (tert-butoxycarbonyl)-L-phenylalanylglycyl-L-proline (3.44 mg, 0.00820 mmol).

$$\text{ESI-MS(+)} = 420.3$$

(Example 3)

<Synthesis of tert-butyl=((S)-1-((2-((S)-2-(((S)-1-(((S)-1-((S)-1-((S)2-carbamoylpyrrolidin-1-yl)-1-oxopropan-2-yl)amino)-1-oxo-3-phenylpropan-2-yl)amino)-4-methyl-1-oxopentan-2-yl)carbamoyl)pyrrolidin-1-yl)-2-oxoethyl)amino)-1-oxo-3-phenylpropan-2-yl) carbamate>

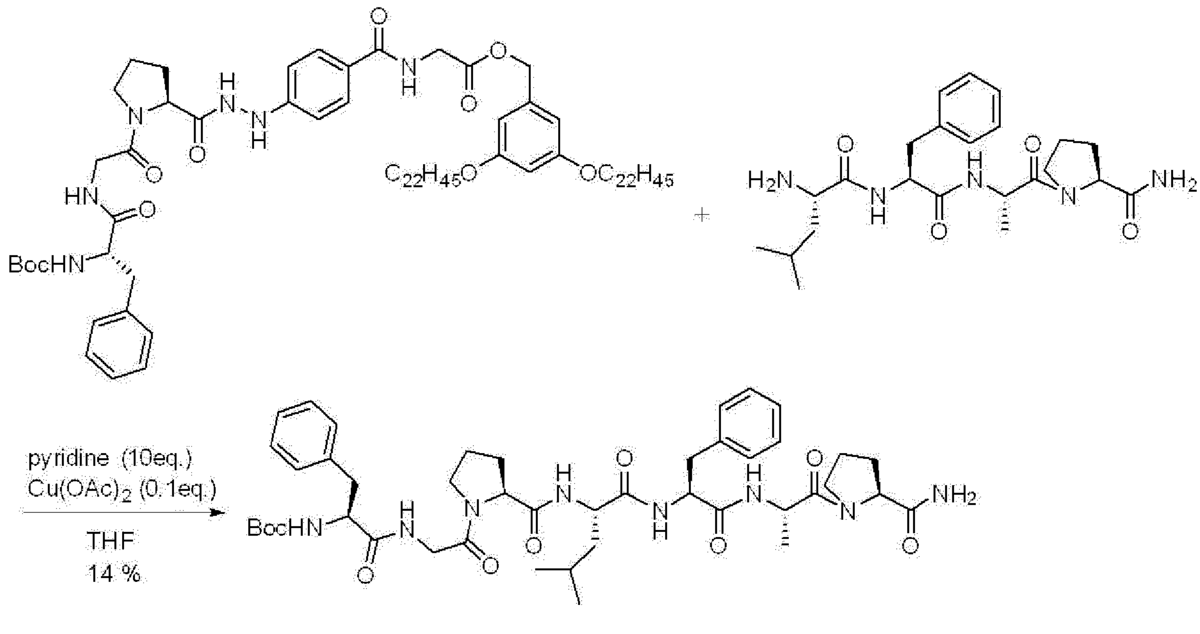


A TFA salt (12.0 mg, 0.0215 mmol) of (S)-1-(L-leucyl-L-phenylalanyl-L-alanyl)pyrrolidine-2-carboxamide, pyridine (15 μL, 0.185 mmol), N-methylmorpholine (10.2 μL, 0.0244 mmol), and copper acetate monohydrate (0.37 mg, 0.00185 mmol) were added to a tetrahydrofuran solution (740 μL) of 3,5-bis(docosyloxy)benzyl=(4-(2-((tert-butoxycarbonyl)-L-phenylalanylglycyl-L-prolyl)hydrazinyl)benzoyl) glycinate (25.0 mg, 0.0185 mmol), and the mixture was stirred at room temperature for 2 hours. The solid precipitated by an addition of methanol was separated and removed by

centrifugation to obtain a supernatant including the target peptide. The solvent was distilled off under reduced pressure, and purification by HPLC was performed to obtain tert-butyl=((S)-1-((2-((S)-2-(((S)-1-(((S)-1-((S)-1-((S)2-carbamoylpyrrolidin-1-yl)-1-oxopropan-2-yl)amino)-1-oxo-3-phenylpropan-2-yl)amino)-4-methyl-1-oxopentan-2-yl)carbamoyl)pyrrolidin-1-yl)-2-oxoethyl)amino)-1-oxo-3-phenylpropan-2-yl) carbamate (2.19 mg, 0.00259 mmol).

$$\text{ESI-MS(-)} = 845.4$$

(Comparative Example 1: formation of diketopiperazine compound)

<Synthesis of 1-((9H-fluoren-9-yl)methyl)=2-(3,5-bis(docosyloxy)benzyl)=(S)-pyrrolidine-1,2-dicarboxylate>

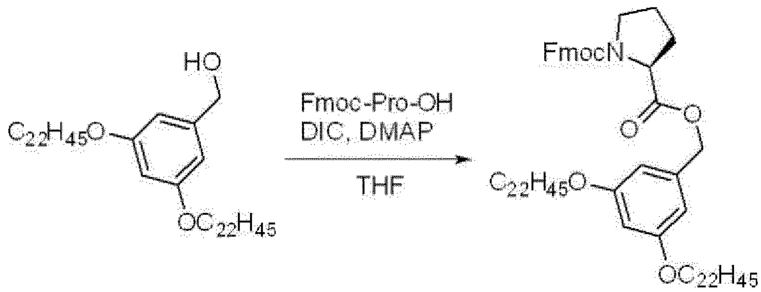


N-[(9H-fluoren-9-ylmethoxy)carbonyl]-L-proline (16.7 mg, 0.0495 mmol), N,N'-diisopropylcarbodiimide (7.75 μL, 0.0495 mmol), and N,N-dimethyl-4-aminopyridine (0.81 mg, 0.0066 mmol) were added to a tetrahydrofuran solution (660 μL) of (3,5-bis(docosyloxy)phenyl)methanol (25.0 mg, 0.0330 mmol), and the mixture was stirred at room temperature overnight. The solid precipitated by adding acetonitrile was recovered by centrifugation to obtain 1-((9H-fluoren-9-yl)methyl)=2-(3,5-bis(docosyloxy)benzyl)=(S)-pyrrolidine-1,2-dicarboxylate (33.3 mg, 0.0309 mmol). The content of the target product was approximately 95 mol% in terms of NMR, proline was not introduced the target product, and approximately 5 mol% of the remaining raw material was mixed in the target product.

<Synthesis of 3,5-bis(docosyloxy)benzyl=(((9H-fluoren-9-yl)methoxy)carbonyl)glycyl-L-prolinate>

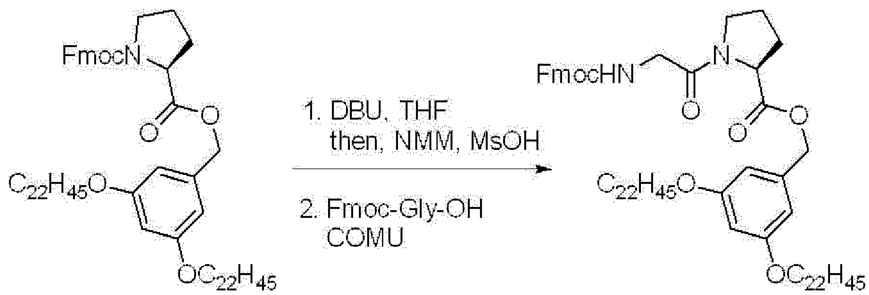


Diazabicycloundecene (65.8 μL, 0.438 mmol) was added to a tetrahydrofuran solution (557 μL) of 1-((9H-fluoren-9-yl)methyl)=2-(3,5-bis(docosyloxy)benzyl)=(S)-pyrrolidine-1,2-dicarboxylate (30.0 mg, 0.0279 mmol), and the mixture was stirred at room temperature for 30 minutes. N-methylmorpholine (6.15 μL, 0.0557 mmol) and methanesulfonic acid (3.74 μL, 0.0571 mmol) were added thereto, and the mixture was stirred at room temperature for 10 minutes. N-[(9H-fluoren-9-ylmethoxy)carbonyl] glycine (10.4 mg, 0.0348 mmol) and cyano-2-ethoxy-2-oxoethylidenaminooxy)dimethylamino-morpholino-carbenium hexafluorophosphate (14.9 mg, 0.0348 mmol) were added to the mixture, and the mixture was stirred at room temperature for 1 hour. The solid precipitated by adding acetonitrile was recovered by centrifugation to obtain 3,5-bis(docosyloxy)benzyl=(((9H-fluoren-9-yl)methoxy)carbonyl)glycyl-L-prolinate (23.0 mg, 0.0203 mmol).

<Synthesis of 3,5-bis(docosyloxy) benzyl=glycyl-L-prolinate ((S)-hexahydropyrrolo[1,2-a]pyrazine-1,4-dione was obtained)>

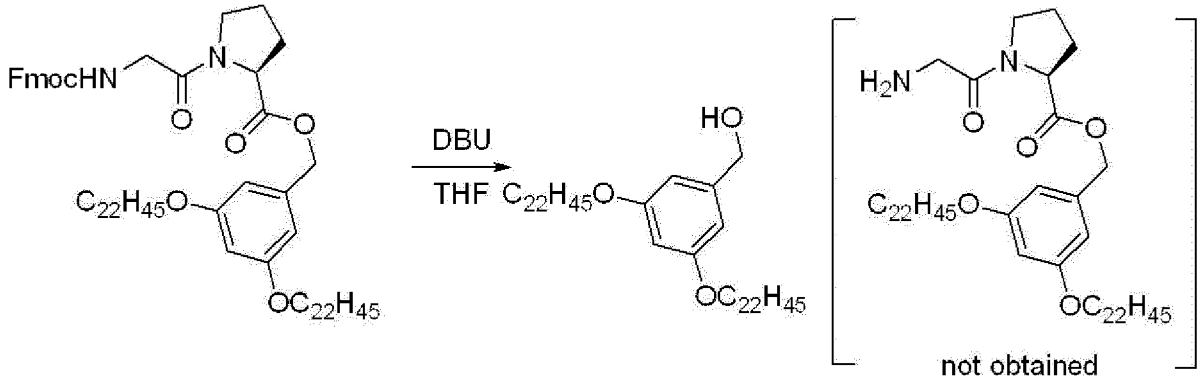


Diazabicycloundecene (6.10 μL, 0.0406 mmol) was added to a tetrahydrofuran solution (405 μL) of 3,5-bis(docosyloxy)benzyl=(((9H-fluoren-9-yl)methoxy)carbonyl)glycyl-L-prolinate (23.0 mg, 0.0203 mmol), and the mixture was stirred at room temperature for 1 hour. In a case where the solid precipitated by adding acetonitrile was recovered by centrifugation, 3,5-bis(docosyloxy)phenyl)methanol (14.2 mg, 0.0188 mmol), which was formed by a cyclization reaction (diketopiperazine formation) of 3,5-bis(docosyloxy) benzyl=glycyl-L-prolinate, was obtained, and as a result, the desired 3,5-bis(docosyloxy) benzyl=glycyl-L-prolinate was not obtained.

(Comparative Example 2: nucleophilic substitution reaction by peptide in solid phase)

<Synthesis of hydrazinobenzoyl AM NovaGel in which resin was bonded to C terminal of (tert-butoxycarbonyl)-L-phenylalanylglycyl-L-proline>

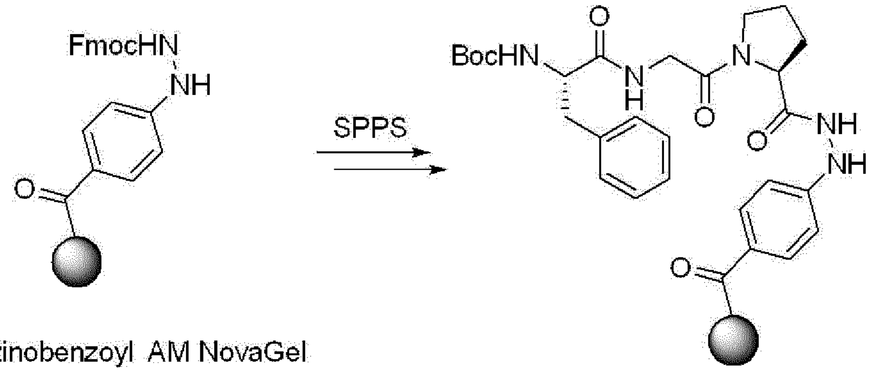


4-Fmoc-hydrazinobenzoyl AM NovaGel

Solid phase peptide synthesis (SPPS) was performed using an automatic peptide synthesizer (SYRO I, manufactured by Biotage Ltd.). 4-Fmoc-hydrazinobenzoyl AM NovaGel (246 mg, 0.61 mmol/g; black sphere represents a bonding position to a solid phase), an N-methylpyrrolidone (NMP) solution of Fmoc amino acid (0.5 mol/L), an NMP solution of ethyl cyano-hydroxyimino-imino acetate (1.0 mol/L), an NMP solution of diisopropylcarbodiimide (1.0 mol/L), and an NMP solution of piperidine (40 %v/v) were set in the synthesizer, and the synthesis was performed according to the manual. Fmoc deprotection (room temperature, 20 minutes), washing with NMP, condensation of Fmoc amino acid (40°C, 1 hour), and washing with NMP were set at one cycle, and by repeating this cycle, the peptide chain was extended. After completion of the extension, the resin was washed with dichloromethane, and then the solvent was distilled off under reduced pressure to obtain 265 mg of a resin.

<Synthesis of tert-butyl=((S)-1-((2-((S)-2-(((S)-1-(((S)-1-((S)-1-((S)-2-carbamoylpyrrolidin-1-yl)-1-oxopropan-2-yl)amino)-1-oxo-3-phenylpropan-2-yl)amino)-4-methyl-1-oxopentan-2-yl)carbamoyl)pyrrolidin-1-yl)-2-oxoethyl)amino)-1-oxo-3-phenylpropan-2-yl) carbamate>

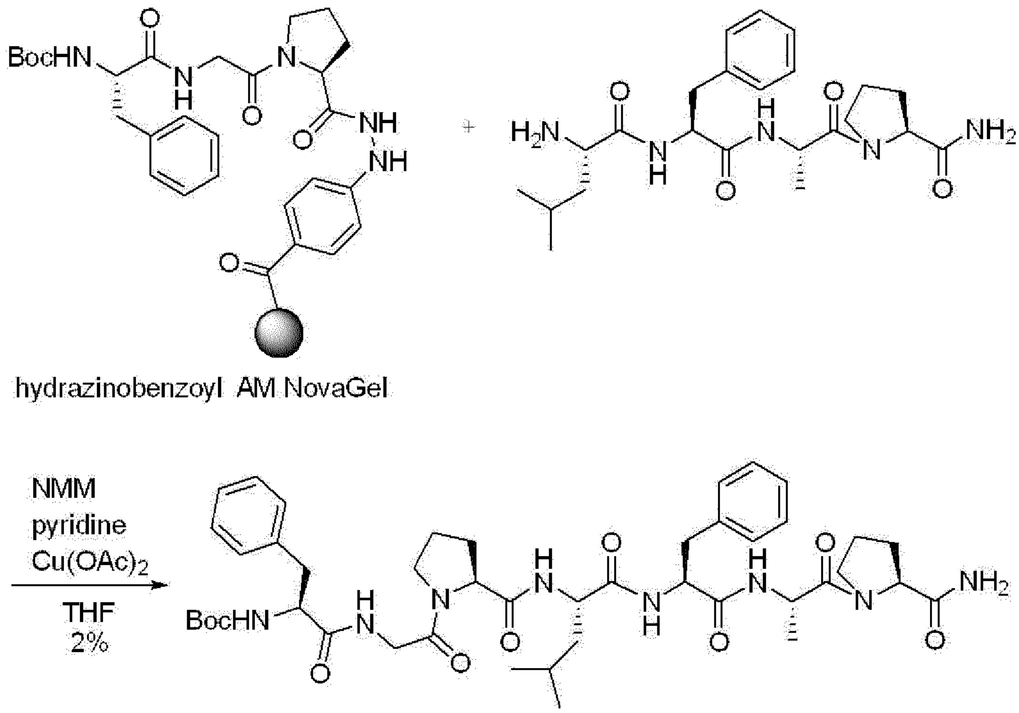


Tetrahydrofuran (740 μL), a TFA salt (12.0 mg, 0.0215 mmol) of (S)-1-(L-leucyl-L-phenylalanyl-L-alanyl)pyrrolidine-2-carboxamide, pyridine (15 μL, 0.185 mmol), N-methylmorpholine (10.2 μL, 0.0244 mmol), and copper acetate monohydrate (0.37 mg, 0.00185 mmol) were added to the hydrazinobenzoyl AM NovaGel in which the resin was bonded to a C terminal of (tert-butoxycarbonyl)-L-phenylalanylglycyl-L-proline (32.7 mg, 0.0185 mmol, based on resin loading), and the mixture was stirred at room temperature for 2 hours. The resin was filtered off, the resin was washed twice with methanol, and the peptide solution was recovered. The solvent was distilled off under reduced pressure, and purification by HPLC was performed to obtain tert-butyl=((S)-1-((2-((S)-2-(((S)-1-(((S)-1-((S)-1-((S)-2-carbamoylpyrrolidin-1-yl)-1-oxopropan-2-yl)amino)-1-oxo-3-phenylpropan-2-yl)amino)-4-methyl-1-oxopentan-2-yl)carbamoyl)pyrrolidin-1-yl)-2-oxoethyl)amino)-1-oxo-3-phenylpropan-2-yl) carbamate (0.33 mg, 0.00039 mmol).

(Comparative Example 3: loading of amino acid into fluorene-type linker)

<Synthesis of 2-((12-(docosyloxy)dodecyl)oxy)-9-(3-fluorophenyl)-9H-fluoren-9-yl(((9H-fluoren-9-yl)methoxy)carbonyl)-L-prolinate>

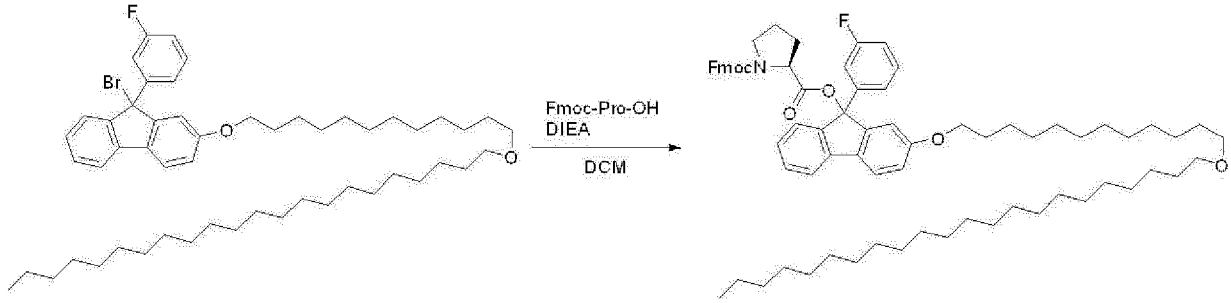

9-Bromo-2-((12-(docosyloxy)dodecyl)oxy)-9-(3-fluorophenyl)-9H-fluorene synthesized by the method described in WO2010/104169A was used.

N-[(9H-fluoren-9-ylmethoxy)carbonyl]-L-proline (59.7 mg, 0.177 mmol) and diisopropylethylamine (DIEA, 61.7 μL, 0.354 mmol) were added to a dichloromethane solution (590 μL) of 9-Bromo-2-((12-(docosyloxy)dodecyl)oxy)-9-(3-fluorophenyl)-9H-fluorene (100 mg, 0.118 mmol) at room temperature, and the mixture was stirred at room temperature overnight. In a case where the solid precipitated by adding acetonitrile was recovered by centrifugation, and NMR was confirmed, formation of the target product 2-((12-(docosyloxy)dodecyl)oxy)-9-(3-fluorophenyl)-9H-fluoren-9-yl(((9H-fluoren-9-yl)methoxy)carbonyl)-L-prolinate was not confirmed.

(Comparative Example 4: loading of amino acid into fluorene-type linker, heating conditions, and increasing reagent amount)

<Synthesis of 2-((12-(docosyloxy)dodecyl)oxy)-9-(3-fluorophenyl)-9H-fluoren-9-yl=(((9H-fluoren-9-yl)methoxy)carbonyl)-L-prolinate>

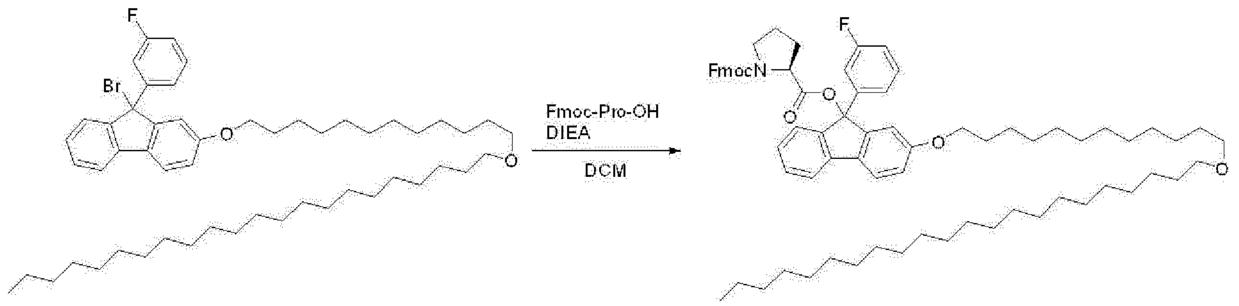


N-[(9H-fluoren-9-ylmethoxy)carbonyl]-L-proline (398 mg, 1.18 mmol) and diisopropylethylamine (206 μL, 1.18 mmol) were added to a dichloromethane solution (6.0 mL) of 9-Bromo-2-((12-(docosyloxy)dodecyl)oxy)-9-(3-fluorophenyl)-9H-fluorene (500 mg, 0.59 mmol) at room temperature, and the mixture was stirred at 50°C for 3 hours and 30 minutes. N-[(9H-fluoren-9-ylmethoxy)carbonyl]-L-proline (199 mg, 0.59 mmol) and diisopropylethylamine (103 μL, 0.59 mmol) were added thereto at room temperature, and the mixture was stirred at 50°C for 4 hours. In a case where the solid precipitated by adding acetonitrile was recovered by centrifugation, and NMR was confirmed, formation of the target product 2-((12-(docosyloxy)dodecyl)oxy)-9-(3-fluorophenyl)-9H-fluoren-9-yl(((9H-fluoren-9-yl)methoxy)carbonyl)-L-prolinate was confirmed, but raw materials and a compound which seemed to be a decomposition product thereof were also observed. Therefore, separation by silica gel column chromatography was performed to obtain 2-((12-(docosyloxy)dodecyl)oxy)-9-(3-fluorophenyl)-9H-fluoren-9-yl=(((9H-fluoren-9-yl)methoxy)carbonyl)-L-prolinate (73.0 mg).

<Synthesis of 2-((12-(docosyloxy)dodecyl)oxy)-9-(3-fluorophenyl)-9H-fluoren-9-yl=(((9H-fluoren-9-yl)methoxy)carbonyl)glycyl-L-prolinate>

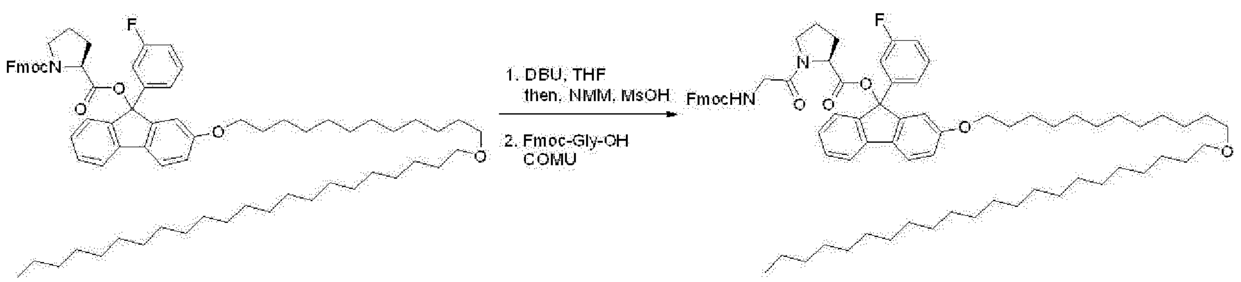


Diazabicycloundecene (15.2 μL, 0.101 mmol) was added to a tetrahydrofuran solution (504 μL) of 2-((12-(docosyloxy)dodecyl)oxy)-9-(3-fluorophenyl)-9H-fluoren-9-yl=(((9H-fluoren-9-yl)methoxy)carbonyl)-L-prolinate (55.7 mg, 0.0504 mmol), and the mixture was stirred at room temperature for 20 minutes. N-methylmorpholine (11.1 μL, 0.101 mmol) and methanesulfonic acid (6.78 μL, 0.103 mmol) were added thereto, and the mixture was stirred at room

temperature for 30 minutes. N-[(9H-fluoren-9-ylmethoxy)carbonyl]-glycine (18.7 mg, 0.0630 mmol) and cyano-2-ethoxy-2-oxoethylidenaminooxy)dimethylamino-morpholino-carbenium hexafluorophosphate (27.0 mg, 0.0630 mmol) were added to the mixture, and the mixture was stirred at room temperature for 30 minutes. The solid precipitated by adding acetonitrile was recovered by centrifugation to obtain 2-((12-(docosyloxy)dodecyl)oxy)-9-(3-fluorophenyl)-9H-fluoren-9-yl=(((9H-fluoren-9-yl)methoxy)carbonyl)glycyl-L-prolinate (24.3 mg, 0.0209 mmol).

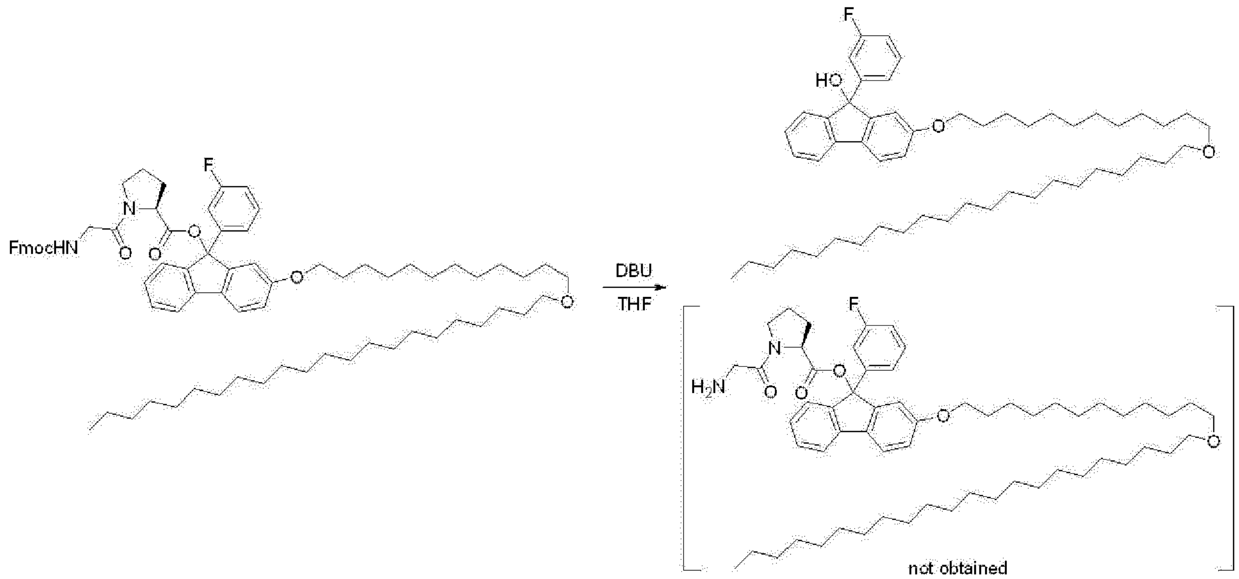


Diazabicycloundecene (4.8 μL, 0.0319 mmol) was added to a tetrahydrofuran solution (320 μL) of 2-((12-(docosyloxy)dodecyl)oxy)-9-(3-fluorophenyl)-9H-fluoren-9-yl=(((9H-fluoren-9-yl)methoxy)carbonyl)glycyl-L-prolinate (18.5 mg, 0.0159 mmol), and the mixture was stirred at room temperature for 20 minutes. In a case where the solid precipitated by adding acetonitrile was recovered by centrifugation, only 2-((12-(docosyloxy)dodecyl)oxy)-9-(3-fluorophenyl)-9H-fluorene-9-ol (8.5 mg, 0.0108 mmol), which was formed from a cyclization reaction (diketopiperazine reaction) of 2-((12-(docosyloxy)dodecyl)oxy)-9-(3-fluorophenyl)-9H-fluoren-9-yl-glycyl-L-prolinate, was obtained.

(Example 4)

<Synthesis of tert-butyl=2-(4-(didocosylcarbamoyl)phenyl)hydrazine-1-carboxylate>

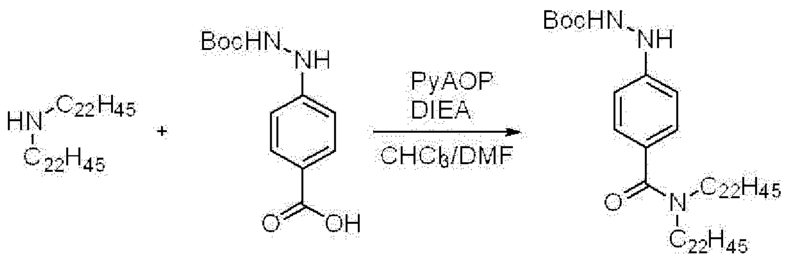


(7-Azabenzotriazol-1-yloxy)tripyrrolidinophosphonium hexafluorophosphate (PyAOP, 5.21 g, 10.0 mmol) and DIEA (3.49 mL, 20.0 mmol) were added to a chloroform solution (50 mL) of dioctadecylamine (2.61 g, 5.00 mmol) and 4-(2-(tert-butoxycarbonyl)hydrazinyl)benzoic acid (2.52 g, 10.0 mmol) at room temperature, and the mixture was stirred for 1.5 hours. The solid precipitated by adding acetonitrile was collected by filtration to obtain tert-butyl=2-(4-(didocosylcarbamoyl)phenyl)hydrazine-1-carboxylate (3.78 g, 4.914 mmol).

<Synthesis of (9H-fluoren-9-yl)methyl=(S)-2-(1-(2-(4-(didocosylcarbamoyl)phenyl)hydrazinyl)pyrrolidin-2-yl)-2-oxoacetate>

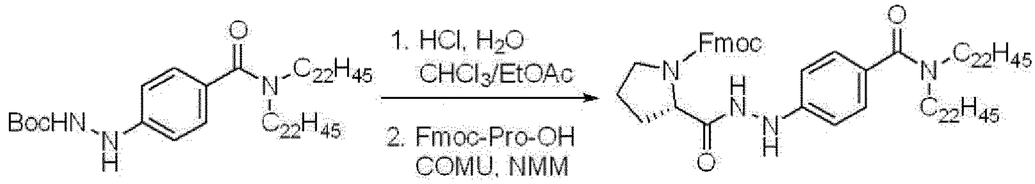


Water (623 μL), chloroform (12.5 mL), and an ethyl acetate solution of hydrogen chloride (4 mol/L, 18.7 mL) were added to tert-butyl=2-(4-(didocosylcarbamoyl)phenyl)hydrazine-1-carboxylate (1.25 g, 1.436 mmol) at room temperature, and the mixture was stirred for 30 minutes to remove a Boc group. The solvent was distilled off under reduced pressure to obtain a white solid. Tetrahydrofuran (28.7 mL), Fmoc-Pro-OH (606 mg, 1.80 mmol), NMM (317 μL, 2.87 mmol), and COMU (769 mg, 1.80 mmol) were added thereto, and the mixture was stirred at room temperature for 30 minutes. The solid precipitated by adding acetonitrile was collected by filtration to obtain (9H-fluoren-9-yl)methyl=(S)-2-(1-(2-(4-(didocosylcarbamoyl)phenyl)hydrazinyl)pyrrolidin-2-yl)-2-oxoacetate (1.56 g, 1.28 mmol).

<Synthesis of (9H-fluoren-9-yl)methyl=(S)-(2-(2-(2-(2-(4-(didocosylcarbamoyl)phenyl)hydrazine-1-carbonyl)pyrrolidin-1-yl)-2-oxoethyl)carbamate>

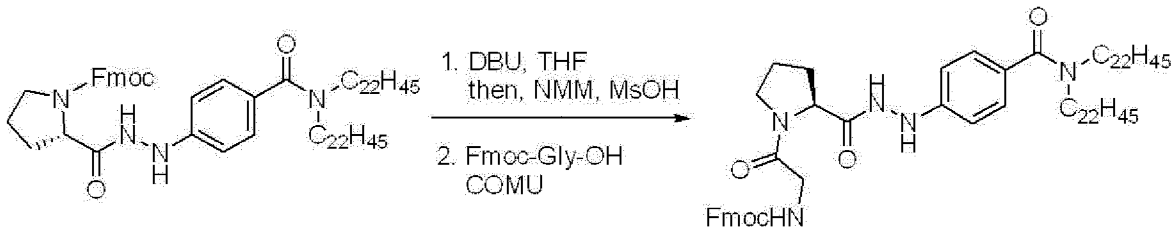


Diazabicycloundecene (190 μL, 1.27 mmol) was added to a tetrahydrofuran solution (12.7 mL) of (9H-fluoren-9-yl)methyl=(S)-2-(1-(2-(4-(didocosylcarbamoyl)phenyl)hydrazinyl)pyrrolidin-2-yl)-2-oxoacetate (688 mg, 0.633 mmol), and the mixture was stirred at room temperature for 20 minutes. NMM (140 μL, 1.27 mmol) and methanesulfonic acid (85 μL, 1.30 mmol) were added thereto, and the mixture was stirred at room temperature for 10 minutes. Fmoc-Gly-OH (235 mg, 0.791 mmol) and COMU (339 mg, 0.791 mmol) were added to the mixture, and the mixture was stirred at room temperature for 30 minutes. The solid precipitated by adding 5% water-containing acetonitrile was collected by filtration to obtain (9H-fluoren-9-yl)methyl=(S)-(2-(2-(2-(2-(4-(didocosylcarbamoyl)phenyl)hydrazine-1-carbonyl)pyrrolidin-1-yl)-2-oxoethyl)carbamate (668 mg, 0.584 mmol).

<Synthesis of tert-butyl=((S)-1-((2-((S)-2-(2-(4-(didocosylcarbamoyl)phenyl)hydrazine-1-carbonyl)pyrrolidin-1-yl)-2-oxoethyl)amino)-1-oxo-3-phenylpropan-2-yl)carbamate>

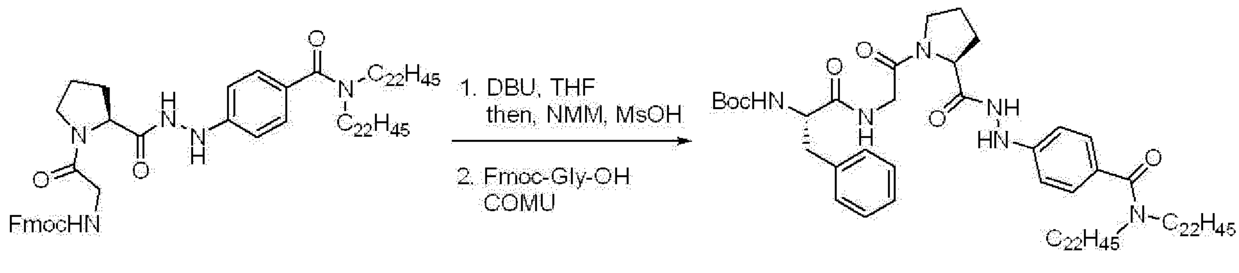

DBU (329 μL, 2.19 mmol) was added to a tetrahydrofuran solution (14.6 mL) of (9H-fluoren-9-yl)methyl=(S)-(2-(2-(2-(2-(4-(didocosylcarbamoyl)phenyl)hydrazine-1-carbonyl)pyrrolidin-1-yl)-2-oxoethyl)carbamate (1.25 g, 1.09 mmol), and the mixture was stirred at room temperature for 20 minutes. NMM (241 μL, 2.19 mmol) and methanesulfonic acid (147 μL, 2.24 mmol) were added thereto, and the mixture was stirred at room temperature for 10 minutes. Boc-Phe-OH (362 mg, 1.37 mmol) and COMU (585 mg, 1.37 mmol) were added to the mixture, and the mixture was stirred at room temperature for 30 minutes. The solid precipitated by adding acetonitrile was collected by filtration to obtain tert-butyl=((S)-1-((2-((S)-2-(2-(4-(didocosylcarbamoyl)phenyl)hydrazine-1-carbonyl)pyrrolidin-1-yl)-2-oxoethyl)amino)-1-oxo-3-phenylpropan-2-yl)carbamate (1.20 g, 1.02 mmol).

<Synthesis of (tert-butoxycarbonyl)-L-phenylalanylglycyl-L-proline>

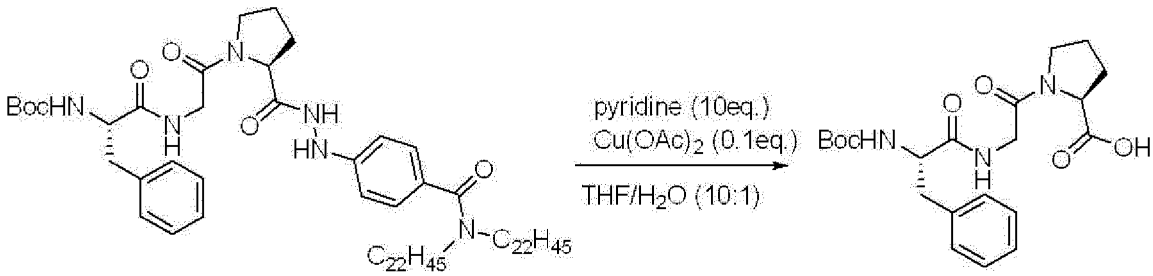


Water (340 μL), pyridine (69.1 μL, 0.855 mmol), and copper acetate monohydrate ($Cu(OAc)_2$, 1.62 mg, 0.00855 mmol) were added to a tetrahydrofuran solution (3.4 mL) of tert-butyl=((S)-1-((2-((S)-2-(2-(4-(didocosylcarbamoyl)phenyl)hydrazine-1-carbonyl)pyrrolidin-1-yl)-2-oxoethyl)amino)-1-oxo-3-phenylpropan-2-yl)carbamate (100 mg, 0.0854 mmol), and the mixture was stirred at room temperature for 2 hours. The solid precipitated by adding methanol was removed by centrifugation to obtain a supernatant including the target peptide compound. The solvent was distilled off under reduced pressure, and then purification by HPLC was performed to obtain (tert-butoxycarbonyl)-L-phenylalanylglycyl-L-proline (6.33 mg).

ESI-MS(+) = 420.3

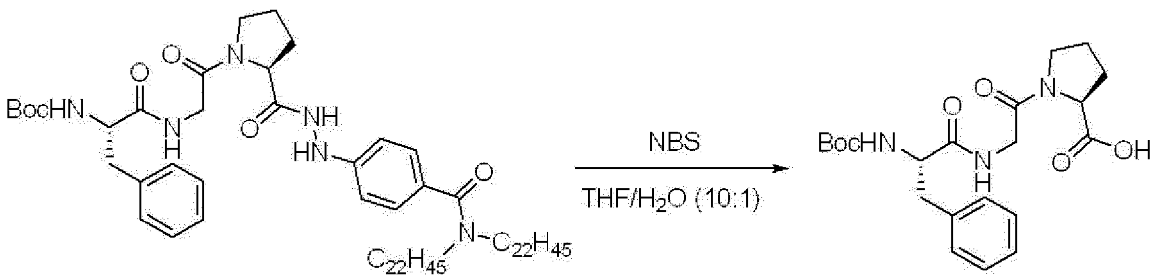


Water (170 μL) and N-bromosuccinimide (NBS, 15.2 mg, 0.0855 mmol) were added to a tetrahydrofuran solution (1.7 mL) of tert-butyl=((S)-1-((2-((S)-2-(2-(4-(didocosylcarbamoyl)phenyl)hydrazine-1-carbonyl)pyrrolidin-1-yl)-2-oxoethyl)amino)-1-oxo-3-phenylpropan-2-yl)carbamate (50 mg, 0.0427 mmol), and the mixture was stirred at room temperature for 2 hours. The solid precipitated by adding methanol was removed by centrifugation to obtain a supernatant including the target peptide compound. The solvent was distilled off under reduced pressure, and then purification by HPLC was performed to obtain (tert-butoxycarbonyl)-L-phenylalanylglycyl-L-proline (0.72 mg).

ESI-MS(+) = 420.3

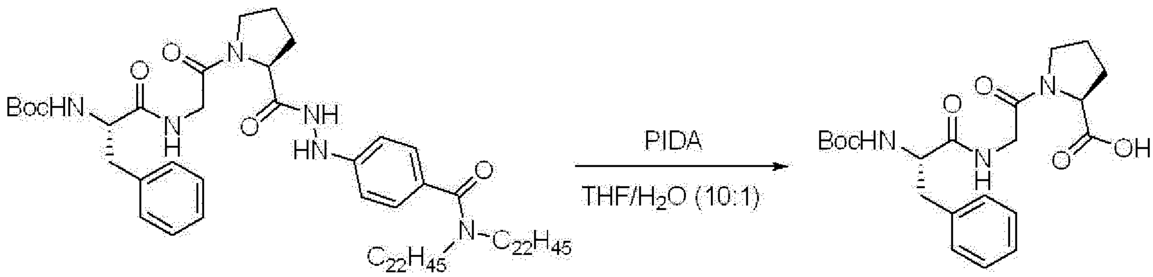


Water (170 μL) and phenyliododiacetate (PIDA, 27.5 mg, 0.0855 mmol) were added to a tetrahydrofuran solution (1.7 mL) of tert-butyl=((S)-1-((2-((S)-2-(2-(4-(didocosylcarbamoyl)phenyl)hydrazine-1-carbonyl)pyrrolidin-1-yl)-2-oxoethyl)amino)-1-oxo-3-phenylpropan-2-yl)carbamate (50 mg, 0.0427 mmol), and the mixture was stirred at room temperature for 2 hours. The solid precipitated by adding methanol was removed by centrifugation to obtain a supernatant including the target peptide compound. The solvent was distilled off under reduced pressure, and then purification by HPLC was performed to obtain (tert-butoxycarbonyl)-L-phenylalanylglycyl-L-proline (6.57 mg).

ESI-MS(+) = 420.3

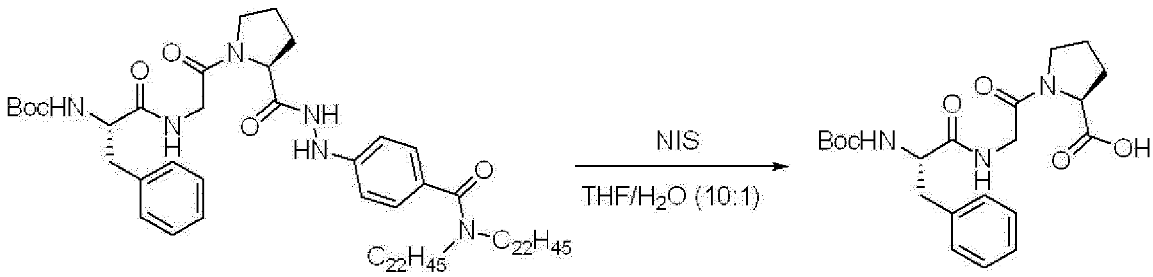


Water (170 μL) and N-broiodosuccinimide (NIS, 19.2 mg, 0.0855 mmol) were added to a tetrahydrofuran solution (1.7 mL) of tert-butyl=((S)-1-((2-((S)-2-(2-(4-(didocosylcarbamoyl)phenyl)hydrazine-1-carbonyl)pyrrolidin-1-yl)-2-oxoethyl)amino)-1-oxo-3-phenylpropan-2-yl)carbamate (50 mg, 0.0427 mmol), and the mixture was stirred at room temperature for 2 hours. The solid precipitated by adding methanol was removed by centrifugation to obtain a supernatant including the target peptide compound. The solvent was distilled off under reduced pressure, and then purification by HPLC was performed to obtain (tert-butoxycarbonyl)-L-phenylalanylglycyl-L-proline (4.82 mg).

ESI-MS(+) = 420.3

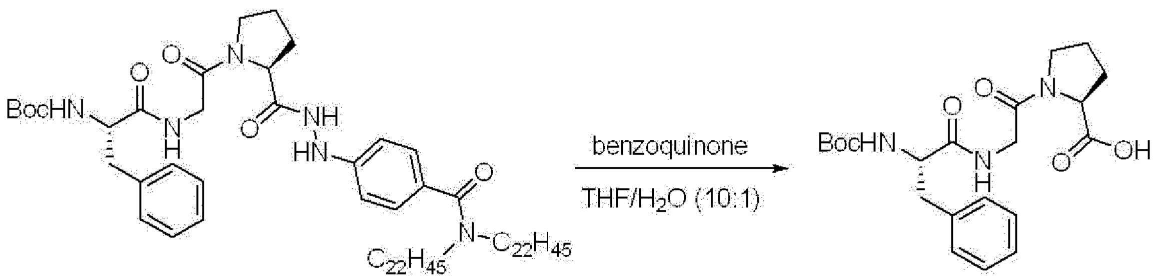


Water (340 μL) and benzoquinone (11.1 mg, 0.103 mmol) were added to a tetrahydrofuran solution (3.4 mL) of tert-butyl((S)-1-((2-((S)-2-(2-(4-(didocosylcarbamoyl)phenyl)hydrazine-1-carbonyl)pyrrolidin-1-yl)-2-oxoethyl)amino)-1-oxo-3-phenylpropan-2-yl)carbamate (100 mg, 0.0854 mmol), and the mixture was stirred at room temperature for 2 hours. After adding sodium sulfite (4.9 mg), the solid precipitated by adding 10% water-containing acetonitrile was removed by centrifugation to obtain a supernatant including the target peptide compound. The solvent was distilled off under reduced pressure, and then purification by HPLC was performed to obtain (tert-butoxycarbonyl)-L-phenylalanylglycyl-L-proline (3.78 mg).

ESI-MS(+) = 420.3

As shown above, the method for producing a peptide compound in Examples using the hydrazine derivative represented by Formula (1) was superior in yield in a case of deprotection as compared with the method for producing a peptide compound in Comparative Examples.

In addition, as shown above, in the method for producing a peptide compound in Examples using the hydrazine derivative represented by Formula (1), the peptide compound could be synthesized in a high yield even during protection and peptide chain extension, and by-production of diketopiperazine compound could be suppressed. In addition, it was also confirmed that the protection of C-terminal amino acid proceeded in good yield without residual raw materials.

The disclosure of Japanese Patent Application No. 2019-165967 filed on September 12, 2019 is incorporated in the present specification by reference.

All documents, patent applications, and technical standards described in the present specification are incorporated herein by reference to the same extent as in a case of being specifically and individually noted that individual documents, patent applications, and technical standards are incorporated by reference.

## Claims

1. A method for producing a peptide compound, comprising:
a step of using a hydrazine derivative represented by Formula (1),

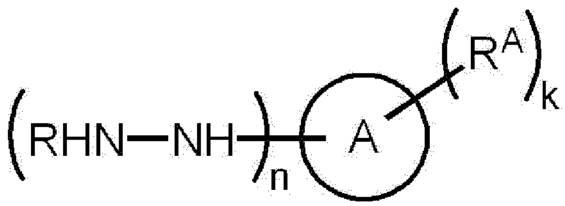

(1)

in Formula (1),

a ring A represents an aromatic hydrocarbon ring or an aromatic heterocyclic ring,
R represents a hydrogen atom, an amino protective group, an amino acid residue, or a peptide residue,
k represents an integer of 1 to 5,
n represents 1 or 2,
$R^A$'s each independently represent an aliphatic hydrocarbon group or an organic group having an aliphatic hydrocarbon group, and
at least one aliphatic hydrocarbon group in k pieces of $R^A$'s has 13 or more carbon atoms.

2. The method for producing a peptide compound according to claim 1,
wherein the step of using the hydrazine derivative represented by Formula (1) is a C-terminal protecting step of protecting a carboxy group of an amino acid compound or a peptide compound with the hydrazine derivative represented by Formula (1).

3. The method for producing a peptide compound according to claim 2,
wherein the amino acid compound or the peptide compound in the C-terminal protecting step is an N-terminal protected amino acid compound or an N-terminal protected peptide compound.

4. The method for producing a peptide compound according to claim 3, further comprising:

an N-terminal deprotecting step of deprotecting an N-terminal of an N-terminal and C-terminal protected amino acid compound or an N-terminal and C-terminal protected peptide compound, which is obtained in the C-terminal protecting step; and
a peptide chain extending step of condensing the N-terminal of a C-terminal protected amino acid compound or a C-terminal protected peptide compound, which is obtained in the N-terminal deprotecting step, with an N-terminal protected amino acid compound or an N-terminal protected peptide compound.

5. The method for producing a peptide compound according to claim 4, further comprising:
a precipitating step of precipitating the N-terminal and C-terminal protected peptide compound obtained in the peptide chain extending step.

**6.** The method for producing a peptide compound according to claim 4, further comprising: an extracting step of adding water to the N-terminal and C-terminal protected peptide compound obtained in the peptide chain extending step to extract and separate impurities into a water phase.

**7.** The method for producing a peptide compound according to claim 5 or 6, further comprising: one or more times in the following order after the precipitating step of the extracting step,

a step of deprotecting the N-terminal of the obtained N-terminal and C-terminal protected peptide compound;
a step of condensing the N-terminal of the obtained C-terminal protected peptide compound with an N-terminal protected amino acid compound or an N-terminal protected peptide compound; and
a step of precipitating the obtained N-terminal and C-terminal protected peptide compound.

**8.** The method for producing a peptide compound according to any one of claims 1 to 7, further comprising: a C-terminal deprotecting step of deprotecting a C-terminal protective group.

**9.** The method for producing a peptide compound according to any one of claims 1 to 8, wherein the ring A is a benzene ring, a naphthalene ring, or a pyridine ring.

**10.** The method for producing a peptide compound according to any one of claims 1 to 9, wherein, in Formula (1), at least two aliphatic hydrocarbon groups in k pieces of $R^A$'s have 13 or more carbon atoms.

**11.** A reagent for forming a protective group comprising: a hydrazine derivative represented by Formula (1a),

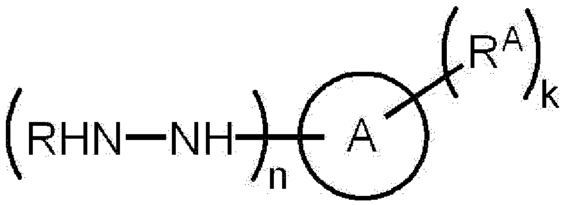
(1a)

in Formula (1a),

a ring A represents an aromatic hydrocarbon ring or an aromatic heterocyclic ring,
R represents a hydrogen atom or an amino protective group,
k represents an integer of 1 to 5,
n represents 1 or 2,
$R^A$'s each independently represent an aliphatic hydrocarbon group or an organic group having an aliphatic hydrocarbon group, and
at least one aliphatic hydrocarbon group in k pieces of $R^A$'s has 13 or more carbon atoms.

**12.** The reagent for forming a protective group according to claim 11, wherein the reagent for forming a protective group is a reagent for forming a protective group of a carboxy group.

**13.** The reagent for forming a protective group according to claim 11 or 12, wherein the reagent for forming a protective group is a C-terminal reagent for forming a protective group of a carboxy group of an amino acid compound, or of a peptide compound.

**14.** The reagent for forming a protective group according to any one of claims 11 to 13, wherein the ring A is a benzene ring, a naphthalene ring, or a pyridine ring.

**15.** The reagent for forming a protective group according to any one of claims 11 to 14, wherein, in Formula (1a), at least two aliphatic hydrocarbon groups in k pieces of $R^A$'s have 13 or more carbon atoms.

**16.** A hydrazine derivative represented by Formula (1b),

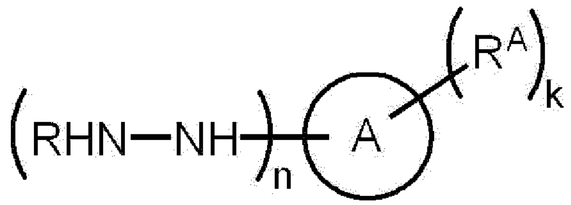

( 1b )

in Formula (1b),

a ring A represents an aromatic hydrocarbon ring or an aromatic heterocyclic ring,
R represents a hydrogen atom or an amino protective group,
k represents an integer of 1 to 5,
n represents 1 or 2,
$R^A$'s each independently represent an aliphatic hydrocarbon group or an organic group having an aliphatic hydrocarbon group, and
at least two aliphatic hydrocarbon groups in k pieces of $R^A$'s have 13 or more carbon atoms.

**INTERNATIONAL SEARCH REPORT**

International application No. PCT/JP2020/034194

A. CLASSIFICATION OF SUBJECT MATTER
C07K 1/06(2006.01)i
FI: C07K1/06
According to International Patent Classification (IPC) or to both national classification and IPC

B. FIELDS SEARCHED
Minimum documentation searched (classification system followed by classification symbols)
C07K1/06

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| A | JP 2008-127331 A (FUJIFILM CORPORATION) 05 June 2008 (2008-06-05) claims, examples | 1-16 |
| A | JP 2005-148108 A (KONICA MINOLTA MEDICAL & GRAPHIC, INC.) 09 June 2005 (2005-06-09) paragraphs [0041]-[0043] | 1-16 |
| A | WO 2010/113939 A1 (AJINOMOTO CO., INC.) 07 October 2010 (2010-10-07) claims, examples | 1-16 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 October 2020 (26.10.2020) | 17 November 2020 (17.11.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application no.
PCT/JP2020/034194

| Patent Documents referred in the Report | Publication Date | Patent Family | Publication Date |
|---|---|---|---|
| JP 2008-127331 A | 05 Jun. 2008 | (Family: none) | |
| JP 2005-148108 A | 09 Jun. 2005 | (Family: none) | |
| WO 2010/113939 A1 | 07 Oct. 2010 | US 2010/0249374 A1 claims, examples | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007034812 A
- WO 2010113939 A
- WO 2010104169 A
- JP 2019165967 A


### Non-patent literature cited in the description


- *Organic Process Research & Development,* 2017, vol. 21 (3), 365-369